(19) Europäisches Patentamt
European Patent Office
Office européen des brevets

(11) Veröffentlichungsnummer: **0 082 108**
**B1**

(12) # EUROPÄISCHE PATENTSCHRIFT

(45) Veröffentlichungstag der Patentschrift: **13.08.86**

(21) Anmeldenummer: **82810414.1**

(22) Anmeldetag: **07.10.82**

(51) Int. Cl.⁴: **C 07 D 251/52**, C 07 D 239/48, C 07 D 251/42, C 07 D 251/18, C 07 D 251/50, A 01 N 47/36

(54) **N-Phenylsulfonyl-N'-pyrimidinyl- und -triazinylharnstoffe.**

(30) Priorität: **13.10.81 CH 6541/81**

(43) Veröffentlichungstag der Anmeldung: **22.06.83 Patentblatt 83/25**

(45) Bekanntmachung des Hinweises auf die Patenterteilung: **13.08.86 Patentblatt 86/33**

(84) Benannte Vertragsstaaten: **AT BE CH DE FR GB IT LI NL**

(56) Entgegenhaltungen:
**EP-A-0 007 687**
**EP-A-0 009 419**
**EP-A-0 023 422**
**EP-A-0 030 139**
**EP-A-0 035 893**
**EP-A-0 044 807**
**EP-A-0 044 808**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Meyer, Willy, Talweg 49, CH- 4125 Riehen (CH)**
Erfinder: **Föry, Werner, Dr., Benkenstrasse 65, CH- 4054 Basel (CH)**
Erfinder: **Gass, Karl, Blumenrain 4, CH- 4465 Magden (CH)**

**EP 0 082 108 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue, herbizid wirksame und pflanzenwuchsregulierende N-Phenylsulfonyl-N'-pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Die erfindungsgemässen N-Phenylsulfonyl-N'-pyrimidinyl- und -triazinylharnstoffe entsprechen der allgemeinen Formel I

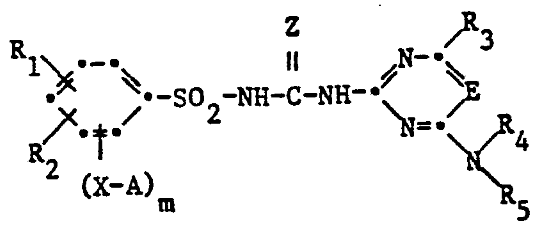 (I),

worin

A einen $C_3$—$C_6$-Alkinylrest, einen durch Halogen, $C_1$—$C_4$-Alkoxy, $C_1$—$C_4$-alkylthio, $C_1$—$C_4$-Alkylsulfinyl, $C_1$—$C_4$-Alkylsulfonyl, $C_1$—$C_4$-Halogenalkoxy, $C_1$—$C_4$-Halogenalkylthio, $C_1$—$C_4$-Halogenalkylsulfinyl oder $C_1$—$C_4$-Halogenalkylsulfonyl substituierten $C_1$—$C_6$-Alkylrest oder einen durch die aufgezählten Reste substituierten $C_2$—$C_6$-Alkenylrest,

E die Methingruppe oder Stickstoff,

X Sauerstoff, Schwefel, eine Sulfinyl- oder Sulfonylbrücke,

Z Sauerstoff oder Schwefel,

m die Zahl eines oder zwei,

$R_1$ Wasserstoff, Halogen, $C_1$—$C_5$-Alkyl, $C_2$—$C_5$-Alkenyl oder einen Rest —Y—$R_6$,

$R_2$ Wasserstoff, Halogen, $C_1$—$C_5$-Alkyl, $C_2$—$C_5$-Alkenyl, $C_1$—$C_4$-Halogenalkyl, oder einen Rest —Y—$R_6$, —$COOR_7$, —$NO_2$ oder —CO—$NR_8$—$R_9$,

$R_3$ Wasserstoff, $C_1$—$C_4$-Alkyl, $C_1$—$C_4$-Alkoxy, $C_1$—$C_4$-Alkylthio, $C_1$—$C_4$-Halogenalkyl, $C_1$—$C_4$-Halogenalkoxy, Halogen oder Alkoxyalkyl mit höchstens 4 Kohlenstoffatomen,

$R_4$ Wasserstoff, Methyl oder Aethyl,

$R_5$ Wasserstoff, $C_1$—$C_4$-Alkyl, $C_1$—$C_3$-Alkoxy, Methoxymethyl, Cyanomethyl oder Cyanoäthyl,

$R_6$ und $R_7$ je $C_1$—$C_5$-Alkyl, $C_2$—$C_5$-Alkenyl oder $C_2$—$C_6$-Alkinyl,

$R_8$ und $R_9$ unabhängig voneinander Wasserstoff, $C_1$—$C_5$-Alkyl, $C_2$—$C_5$-Alkenyl oder $C_2$—$C_6$-Alkinyl und

Y Sauerstoff, Schwefel, eine Sulfinyl- oder Sulfonylbrücke bedeuten, wobei A auch $C_2$—$C_6$-Alkenyl bedeuten kann, wenn X für Sauerstoff steht, sowie den Salzen dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise in den europäischen Patentpublikationen EP—A—9419, EP—A—10560, EP—A—23422 und EP—A—35893 oder im niederländischen Patent Nr. 121788 beschrieben.

In den Definitionen ist unter Alkyl geradkettiges oder verzweigtes Alkyl zu verstehen: z.B.: Methyl, Aethyl, n-Propyl, i-Propyl, die vier isomeren Butyl, n-Amyl, i-Amyl, 2-Amyl, 3-Amyl, n-Hexyl oder i-Hexyl.

Unter Alkoxy ist zu verstehen: Methoxy, Aethoxy, n-Propyloxy, i-Propyloxy und die vier isomeren Butyloxyreste, insbesondere aber Methoxy, Aethoxy oder i-Propoxy.

Beispiele für Alkylthio sind Methylthio, Aethylthio, n-Propylthio, i-Propylthio und n-Butylthio, insbesondere aber Methylthio und Aethylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfinyl sind Methylsulfinyl, Aethylsulfinyl, n-Propylsulfinyl und n-Butylsulfinyl, insbesondere aber Methylsulfinyl und Aethylsulfinyl.

Beispiel für Alkylsulfonyl sind Methylsulfonyl, Aethylsulfonyl, n-Propylsulfonyl und n-Butylsulfonyl, insbesondere aber Methylsulfonyl und Aethylsulfonyl.

Unter Halogen in den Definitionen sowie in Halogenalkyl, -alkoxy, -alkylsulfinyl, -alkylsulfonyl und -alkylthio sind Fluor, Chlor und Brom, vorzugsweise jedoch Fluor und Chlor zu verstehen.

Entsprechend sind unter Halogenalkyl bzw. Halogenalkylteilen von oben definierten Substituenten beispielsweise zu verstehen: Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Alkinylresten in den Definitionen der obigen Symbole entsprechen in der Regel Propargyl, 2-Butinyl, 3-Butinyl, sowie isomere Pentinyl- oder Hexinylreste, vorzugsweise ist der Alkinylrest jedoch durch Propargyl oder 2- oder 3-Butinyl verkörpert.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Aethylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemässen Verbindungen der Formel sind die Verbindungen bevorzugt, in denen
a) X und
b) Z Sauerstoff und
c) m die Zahl eins bedeuten
d) die Reste $R_4$ und $R_5$ zusammen nicht mehr als 4 Kohlenstoffatome enthalten und
e) ein Rest —X—A im Phenylring die 2-Stellung zur Sulfonamidgruppe besetzt.

Durch Kombination der bevorzugten Merkmale ergibt sich eine weiter bevorzugte Gruppe von Verbindungen der Formel I, in denen X und Y Sauerstoff und m die Zahl eins bedeuten, $R_4$ und $R_5$ zusammen nicht mehr als 4 Kohlenstoffatome enthalten und der Rest —X—A im Phenylring die 2-Stellung zur Sulfonamidgruppe besetzt.

Diese bevorzugte Untergruppe zerfällt seinerseits in die beiden folgenden, gleichermassen weiter bevorzugten Gruppen, die Verbindungen enthalten, in denen
α) A für einen $C_1$—$C_4$-Halogenalkylrest steht oder
β) A $C_3$—$C_4$-Alkinyl, $C_2$—$C_6$-Alkenyl oder $C_2$—$C_6$-Halogenalkenyl oder durch $C_1$—$C_4$-Alkoxy, $C_1$—$C_4$-Alkylthio, $C_1$—$C_4$-Alkylsulfinyl oder $C_1$—$C_4$-Alkylsulfonyl substituiertes $C_1$—$C_3$-Alkyl bedeutet.

Eine weitere Bevorzugung innerhalb der Gruppen α und β besteht darin, dass jeweils $R_1$ Wasserstoff und $R_2$ Wasserstoff, Halogen, Methyl oder —COO—$C_1$—$C_3$-Alkyl bedeuten.

Unter diesen Verbindungen sind diejenigen hervorzuheben, in denen $R_1$ und $R_2$ Wasserstoff bedeuten.

Als ganz besonders bevorzugt sind hiervon die Verbindungen anzusehen, in denen $R_3$ Methoxy, Aethoxy, Difluormethoxy, 2,2,2-Trifluoräthoxy, Methyl, Aethyl, Fluormethyl oder Chlormethyl, $R_4$ Wasserstoff, Methyl oder Aethyl und $R_5$ Methyl oder Aethyl bedeuten.

Als bevorzugte Einzelverbindungen sind zu nennen:
N-(2-Difluormethoxyphenyl-sulfonyl)-N'-(4-dimethylamino-6-methoxy-1,3,5-triazin-2-yl)-harnstoff,
N-(2-Difluormethoxyphenyl-sulfonyl)-N'-(4-dimethylamino-6-methoxypyrimidin-2-yl)-harnstoff und
N-(2-Difluormethoxyphenyl-sulfonyl)-N'-(4-methylamino-6-methoxypyrimidin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Phenylsulfonamid der Formel II

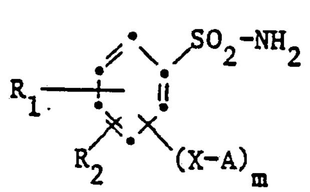
(II),

worin A, $R_1$, $R_2$, X und m die unter Formel I gegebenen Bedeutung haben, in Gegenwart einer Base mit einem N-Pyrimidinyl- oder -Triazinylcarbamat der Formel III

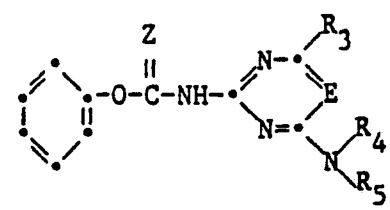
(III),

worin E, $R_3$, $R_4$, $R_5$ und Z die unter Formel I gegebene Bedeutung haben, umsetzt.

Nach einem zweiten Verfahren gelangt man zu Verbindungen der Formel I, indem man ein Phenylsulfonylisocyanat oder -isothiocyanat der Formel IV

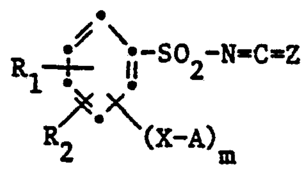

(IV),

worin A, $R_1$, $R_2$, m, X und Z die unter Formel I gegebene Bedeutung haben, gegebenenfalls in Gegenwart einer Base, mit einem Amin der Formel V

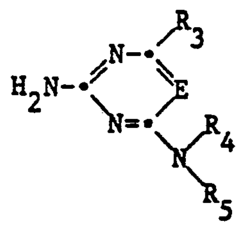

(V),

worin E, $R_3$, $R_4$ und $R_5$ die unter Formel I gegebene Bedeutung haben, umsetzt.

Nach einem weiteren Verfahren werden die Verbindungen der Formel I hergestellt, indem man ein Sulfonamid der oben angegebenen Formel II gegebenenfalls in Gegenwart einer Base mit einem Isocyanat oder Isothiocyanat der Formel VI

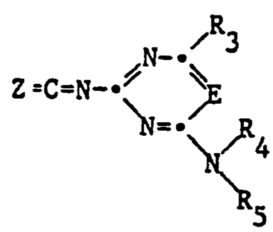

(VI),

worin E, $R_3$, $R_4$, $R_5$ und Z die unter Formel I gegebene Bedeutung haben, umsetzt.

Weiterhin kann man die Verbindungen der Formel I auch erhalten, indem man ein N-Phenylsulfonylcarbamat der Formel VII

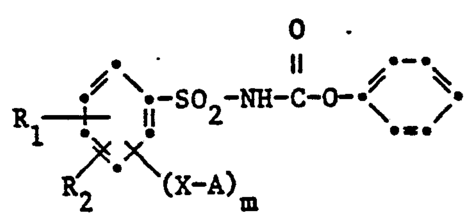

(VII),

worin A, $R_1$, $R_2$, m und X die unter Formel I gegebene Bedeutung haben, mit einem Amin der oben angegebenen Formel V umsetzt.

Schliesslich werden die Verbindungen der Formel I auch hergestellt, indem man einen Sulfonylharnstoff der Formel VIII

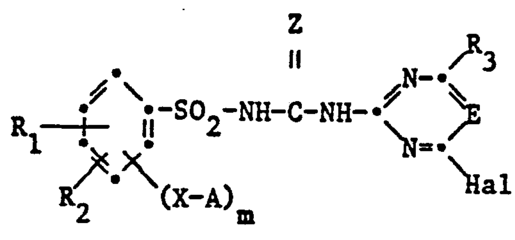

(VIII),

worin A, E, $R_1$, $R_2$, $R_3$, X, Z und m die unter Formel I gegebene Bedeutung haben und Hal für Chlor oder Brom steht, mit einem Amin der Formel IX

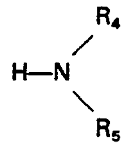

(IX),

worin $R_4$ und $R_5$ die unter Formel I angegebene Bedeutung haben, umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Die Ausgangsstoffe der Formel II, IV, VII, VIII und ortho-substituierte Hydroxyphenyl- resp. substituierte ortho-Hydroxyphenylsulfonamide der Formel X,

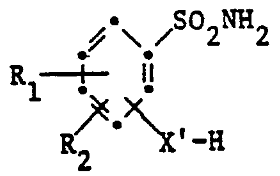

(X),

worin $R_1$ und $R_2$ die unter Formel I angegebene Bedeutung haben und X' für Sauerstoff oder Schwefel steht, als Ausgangsprodukte bestimmter Sulfonamid-Vertreter der Formel II, sind aus der europäischen Patentanmeldung Nr. 44 807 bekannt.

Die Ausgangsmaterialien der Formeln III und VI sind zum Teil bekannt. Neue Verbindungen der Formeln III und VI können nach bekannten Methoden aus entsprechenden Verbindungen der Formel V erhalten werden.

Neue Fluoralkoxy-aminopyrimidine und -triazine der Formel V und ihre Herstellung sowie die Herstellung entsprechender Verbindungen der Formeln III und VI daraus werden in der Europäischen Patentanmeldung EP—A—70 804 beschrieben. Andere Amine der Formel V sind bekannt und zum Teil im Handel erhältlich oder sie können nach bekannten Methoden hergestellt werden, siehe "The Chemistry of Heterocyclic Compounds" Band XIV, Interscience Publishers, New York, London.

Durch Umsetzung von Aminen der Formel V mit Oxalylchlorid in chlorierten Kohlenwasserstoffen als Lösungsmittel, lassen sich Isocyanate der Formel VI herstellen.

Die Ausgangsmaterialien der Formel IX sind bekannt oder können nach bekannten Methoden hergestellt werden.

Die Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen −20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Aether, aromatischen Kohlenwasserstoffen oder chlorierten Kohlenwasserstoffen gereinigt werden.

Die Wirkstoffe der Formel I sind stabile Verbindungen. Ihre Handhabung bedarf keine vorsorglicher Massnahmen.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Zuckerrohr, Getreide, Baumwolle, Soja, Mais und Reis befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei — im Vergleich zu anderen Herbiziden und Wuchsregulatoren — sehr geringen Aufwandmengen.

Die Verbindungen der Formel I haben ausserdem starke pflanzenwuchsregulierende Eigenschaften, die sich in einer Ertragssteigerung von Kulturpflanzen oder Erntegut auswirken kann. Viele Verbindungen

der Formel I zeigen darüberhinaus eine konzentrationsabhängige pflanzenwuchshemmende Wirkung. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei monokotylen Pflanzen, z.B. Gräsern oder auch Kulturpflanzen wie Getreide, ist eine Hemmung des vegetativen Wachstums manchmal gewünscht und vorteilhaft. Eine derartige Wuchshemmung ist unter anderem bei Gräsern von wirtschaftlichem Interesse, denn dadurch kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Unknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen. Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorganulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind

auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8—22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4—14)-Aethylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979. Sisley and Wood, "Encyclopedia of Surface Active Agents". Chemical Publishing Co., Inc. New York, 1964.

Die pestiziden Zubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

<u>Emulgierbare Konzentrate</u>

| | |
|---|---|
| Aktiver Wirkstoff: | 1 bis 20%, bevorzugt 5 bis 10% |
| oberflächenaktives Mittel: | 5 bis 30%, vorzugsweise 10 bis 20% |
| flüssiges Trägermittel: | 50 bis 94%, vorzugsweise 70 bis 85%. |

<u>Stäube</u>

| | |
|---|---|
| Aktiver Wirkstoff: | 0,1 bis 10%, vorzugsweise 0,1 bis 1% |
| festes Trägermittel: | 99,9 bis 90%, vorzugsweise 99,9 bis 99%. |

<u>Suspension-Konzentrate</u>

| | |
|---|---|
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |

Benetzbare Pulver

| | |
|---|---|
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermittel: | 5 bis 95%, vorzugsweise 15 bis 90%. |

Granulate

| | |
|---|---|
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,001 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Herstellungsbeispiele:

Beispiel 1

N-(2-Difluormethoxyphenyl-sulfonyl)-N'-(4-dimethylamino-6-methoxy-1,3,5-triazin-2-yl)-harnstoff.

a) Zur Suspension von 80,3 g 2-Amino-4-chlor-6-methoxy-1,3,5-triazin in 400 ml absolutem Dioxan lässt man in 10 Minuten 124,6 g 2-Difluormethoxy-sulfonylisocyanat in 100 ml Dioxan zutropfen. Die Reaktionsmischung wird für 3 Stunden auf 90—100° erwärmt. Durch Abkühlen und Einengen der Reaktionslösung erhält man 180 g N-(2-Difluormethoxyphenyl-sulfonyl)-N'-(4-chlor-6-methoxy-1,3,5-triazin-2-yl)-harnstoff, Smp. 167—168°.

b) In eine Lösung von 8,2 g N-(2-Difluormethoxyphenyl-sulfonyl)-N'-(4-chlor-6-methoxy-1,3,5-triazin-2-yl)-harnstoff in 80 ml Dioxan werden unter Kühlung bei 20—30°C 3,0 g Dimethylamin eingeleitet. Nachdem die entstandene Suspension für weitere 30 Minuten gerührt worden ist, wird sie in 500 ml 0,1 N-Salzsäure aufgenommen. Das ausgefallene Produkt wird durch Filtration abgetrennt. Ausbeute: 7,7 g N-(2-Difluormethoxyphenyl-sulfonyl)-N'-(4-dimethylamino-6-methoxy-1,3,5-triazin-2-yl)-harnstoff, Smp. 171—172°.

Beispiel 2

N-(2-Difluormethoxyphenyl-sulfonyl)-N'-(4-tert.-butylamino-6-methoxy-1,3,5-triazin-2-yl)-harnstoff.

Eine Mischung von 5,0 g 2-Difluormethoxyphenyl-sulfonylisocyanat und 4,0 g 2-Amino-4-tert.-butylamino-6-methoxy-1,3,5-triazin werden in 60 ml absolutem Dioxan für 3 Stunden bei 70—80°C gerührt. Nach Filtration des heissen Reaktionsgemisches über Aktivkohle kristallisieren beim Abkühlen der Lösung 6,9 g N-(2-Difluormethoxyphenyl-sulfonyl)-N'-(4-tert.-butylamino-6-methoxy-1,3,5-triazin-2-yl)-harnstoff aus, Smp. 213—214°C.

In analoger Weise erhält man die in den anschliessenden Tabellen aufgelisteten Endprodukte.

TABELLE 1

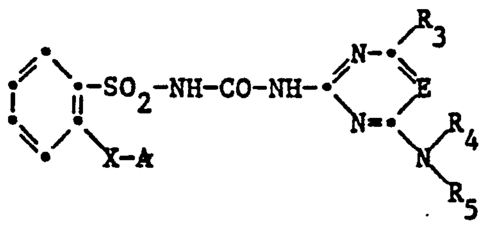

| Nr. | A | X | E | $R_3$ | $R_4$ | $R_5$ | Smp. °C |
|---|---|---|---|---|---|---|---|
| 1 | $CHF_2$ | O | CH | $OCH_3$ | $CH_3$ | $CH_3$ | 202—203 |
| 2 | $CHF_2$ | O | CH | $OCH_3$ | $CH_3$ | $OCH_3$ | |
| 3 | $CHF_2$ | O | CH | $OCH_3$ | $C_2H_5$ | $CH_3$ | |
| 4 | $CHF_2$ | O | CH | $OCH_3$ | $CH_3$ | $-CH_2-CH_2-CN$ | |
| 5 | $CHF_2$ | O | CH | $OCH_3$ | $CH_3$ | $-CH_2-CN$ | |
| 6 | $CHF_2$ | O | CH | $OCH_3$ | $CH_3$ | H | 170—171 |
| 7 | $CHF_2$ | O | CH | $OCH_3$ | H | H | |
| 8 | $CHF_2$ | O | CH | Cl | $CH_3$ | $CH_3$ | 218—219 |
| 9 | $CHF_2$ | O | CH | $CH_3$ | $CH_3$ | $CH_3$ | |
| 10 | $CHF_2$ | O | CH | $CH_3$ | $CH_3$ | $OCH_3$ | |
| 11 | $CHF_2$ | O | CH | $OC_2H_5$ | $CH_3$ | $CH_3$ | |
| 12 | $CHF_2$ | O | CH | $OC_2H_5$ | $CH_3$ | $OCH_3$ | |
| 13 | $CHF_2$ | O | CH | $-OCH_2-CF_3$ | $CH_3$ | $CH_3$ | 193—194 |
| 14 | $CHF_2$ | O | CH | $-OCH_2-CF_3$ | $CH_3$ | $OCH_3$ | |
| 15 | $CHF_2$ | O | CH | $OCH_3$ | $C_2H_5$ | H | |
| 16 | $CHF_2$ | O | N | $OCH_3$ | $CH_3$ | $-CH_2-CN$ | |
| 17 | $CHF_2$ | O | N | $OCH_3$ | $CH_3$ | $-CH_2-CH_2-CN$ | 172—173 |
| 18 | $CHF_2$ | O | N | $OC_2H_5$ | $CH_3$ | $CH_3$ | |
| 19 | $CHF_2$ | O | N | $-OCH_2-CF_3$ | $CH_3$ | $OCH_3$ | |
| 20 | $CHF_2$ | O | N | $-OCH_2-CF_3$ | $CH_3$ | $CH_3$ | 172—173 |
| 21 | $CHF_2$ | O | N | $-OCH_2-CF_3$ | $CH_3$ | $OCH_3$ | |
| 22 | $CHF_2$ | O | N | $CH_3$ | $CH_3$ | $CH_3$ | 193—194 |
| 23 | $CHF_2$ | O | CH | $CH_2Cl$ | $CH_3$ | $CH_3$ | 170—171 |
| 24 | $CHF_2$ | S | CH | $OCH_3$ | $n-C_3H_7$ | $CH_3$ | |
| 25 | $CHF_2$ | S | CH | $OCH_3$ | $CH_3$ | $CH_3$ | |

TABELLE 1 (Fortsetzung)

| Nr. | A | X | E | $R_3$ | $R_4$ | $R_5$ | Smp. °C |
|---|---|---|---|---|---|---|---|
| 26 | $CHF_2$ | S | CH | $OCH_3$ | $C_2H_5$ | $CH_3$ | |
| 27 | $CHF_2$ | S | CH | $OCH_3$ | $CH_3$ | $-CH_2-CH_2-CN$ | |
| 28 | $CHF_2$ | S | CH | $OCH_3$ | $CH_3$ | H | |
| 29 | $CHF_2$ | S | CH | $OCH_3$ | $CH_3$ | $-CH_2-CN$ | |
| 30 | $CHF_2$ | S | CH | $OCH_3$ | $CH_3$ | $CH_3$ | 200—201 |
| 31 | $CHF_2$ | O | CH | $OCH_3$ | $CH_3$ | $OCH_3$ | |
| 32 | $CHF_2$ | O | CH | $OCH_3$ | $CH_3$ | $CH_3$ | |
| 33 | $CHF_2$ | S | N | $OCH_3$ | $CH_3$ | $OCH_3$ | |
| 34 | $CHF_2$ | S | N | $OCH_3$ | $C_2H_5$ | $OCH_3$ | |
| 35 | $CHF_2$ | S | N | $OCH_3$ | $CH_3$ | $OCH_3$ | |
| 36 | $CHF_2$ | S | N | $OCH_3$ | $CH_3$ | $-CH_2-CH_2-CN$ | |
| 37 | $CHF_2$ | S | N | $OCH_3$ | $CH_3$ | $-CH_2-CN$ | |
| 38 | $CHF_2$ | S | N | $OC_2H_5$ | $CH_3$ | $CH_3$ | |
| 39 | $CHF_2$ | S | N | $-OCH_2-CF_3$ | $CH_3$ | $CH_3$ | |
| 40 | $CHF_2$ | S | N | $-OCH_2-CF_3$ | $CH_3$ | $OCH_3$ | |
| 41 | $CHF_2$ | S | N | $OC_2H_5$ | $CH_3$ | $OCH_3$ | |
| 42 | $CHF_2$ | S | CH | Cl | $CH_3$ | $CH_3$ | |
| 43 | $CHF_2$ | S | CH | $CH_3$ | $CH_3$ | $CH_3$ | |
| 44 | $CHF_2$ | S | CH | $CH_3$ | $CH_3$ | $OCH_3$ | |
| 45 | $CHF_2$ | S | CH | $OC_2H_5$ | $CH_3$ | $CH_3$ | |
| 46 | $CHF_2$ | S | CH | $-OCH_2-CF_3$ | $CH_3$ | $OCH_3$ | |
| 47 | $CHF_2$ | S | CH | $-OCH_2-CF_3$ | $CH_3$ | $CH_3$ | |
| 48 | $CHF_2$ | S | CH | $-OC_2H_5$ | $CH_3$ | $OCH_3$ | |
| 49 | $-(CH_2)_2-OCH_3$ | O | CH | $OCH_3$ | $n-C_3H_7$ | $CH_3$ | |
| 50 | $-(CH_2)_2-OCH_3$ | O | CH | $OCH_3$ | $CH_3$ | $OCH_3$ | |
| 51 | $-(CH_2)_2-OCH_3$ | O | CH | $OCH_3$ | $C_2H_5$ | $CH_3$ | |
| 52 | $-(CH_2)_2-OCH_3$ | O | CH | $OCH_3$ | $CH_3$ | $-CH_2-CH_2-CN$ | |
| 53 | $-(CH_2)_2-OCH_3$ | O | CH | $OCH_3$ | $CH_3$ | H | |
| 54 | $-(CH_2)_2-OCH_3$ | O | CH | $OCH_3$ | $CH_3$ | $-CH_2-CN$ | |

TABELLE 1 (Fortsetzung)

| Nr. | A | X | E | $R_3$ | $R_4$ | $R_5$ | Smp. °C |
|---|---|---|---|---|---|---|---|
| 55 | $-(CH_2)_2-OCH_3$ | O | CH | $OCH_3$ | $CH_3$ | $CH_3$ | 202—203 |
| 56 | $-(CH_2)_2-OCH_3$ | S | CH | $OCH_3$ | $CH_3$ | $OCH_3$ | |
| 57 | $-(CH_2)_2-OCH_3$ | S | CH | $OCH_3$ | $CH_3$ | $CH_3$ | |
| 58 | $-(CH_2)_2-OCH_3$ | O | N | $OCH_3$ | $CH_3$ | $OCH_3$ | |
| 59 | $-(CH_2)_2-OCH_3$ | O | N | $OCH_3$ | $C_2H_5$ | $OCH_3$ | |
| 60 | $-(CH_2)_2-OCH_3$ | O | N | $OCH_3$ | $C_2H_5$ | $CH_3$ | |
| 61 | $-(CH_2)_2-OCH_3$ | O | N | $OCH_3$ | $CH_3$ | $-CH_2-CH_2-CN$ | |
| 62 | $-(CH_2)_2-OCH_3$ | O | N | $OCH_3$ | $CH_3$ | $-CH_2-CN$ | |
| 63 | $-(CH_2)_2-OCH_3$ | O | N | $OC_2H_5$ | $CH_3$ | $CH_3$ | |
| 64 | $-(CH_2)_2-OCH_3$ | O | N | $-OCH_2-CF_3$ | $CH_3$ | $CH_3$ | |
| 65 | $-(CH_2)_2-OCH_3$ | O | N | $-OCH_2-CF_3$ | $CH_3$ | $OCH_3$ | |
| 66 | $-(CH_2)_2-OCH_3$ | O | N | $OC_2H_5$ | $CH_3$ | $OCH_3$ | |
| 67 | $-(CH_2)_2-OCH_3$ | O | CH | Cl | $CH_3$ | $CH_3$ | |
| 68 | $-(CH_2)_2-OCH_3$ | O | CH | $CH_3$ | $CH_3$ | $CH_3$ | |
| 69 | $-(CH_2)_2-OCH_3$ | O | CH | $CH_3$ | $CH_3$ | $OCH_3$ | |
| 70 | $-CH_2-CH_2Cl$ | O | CH | $OCH_3$ | $n-C_3H_7$ | $CH_3$ | |
| 71 | $-CH_2-CH_2Cl$ | O | CH | $OCH_3$ | $CH_3$ | $OCH_3$ | |
| 72 | $-CH_2-CH_2Cl$ | O | CH | $OCH_3$ | $C_2H_5$ | $CH_3$ | |
| 73 | $-CH_2-CH_2Cl$ | O | CH | $OCH_3$ | $CH_3$ | $-CH_2-CH_2-CN$ | |
| 74 | $-CH_2-CH_2Cl$ | O | CH | $OCH_3$ | $CH_3$ | H | |
| 75 | $-CH_2-CH_2Cl$ | O | CH | $OCH_3$ | $CH_3$ | $-CH_2-CN$ | |
| 76 | $-CH_2-CH_2Cl$ | O | CH | $OCH_3$ | $CH_3$ | $CH_3$ | |
| 77 | $-CH_2-CH_2Cl$ | S | CH | $OCH_3$ | $CH_3$ | $OCH_3$ | |
| 78 | $-CH_2-CH_2Cl$ | S | CH | $OCH_3$ | $CH_3$ | $CH_3$ | |
| 79 | $-CH_2-CH_2Cl$ | O | N | $OCH_3$ | $CH_3$ | $OCH_3$ | |
| 80 | $-CH_2-CH_2Cl$ | O | N | $OCH_3$ | $C_2H_5$ | $OCH_3$ | |
| 81 | $-CH_2-CH_2Cl$ | O | N | $OCH_3$ | $C_2H_5$ | $CH_3$ | |
| 82 | $-CH_2-CH_2Cl$ | O | N | $OCH_3$ | $CH_3$ | $-CH_2-CH_2-CN$ | |
| 83 | $-CH_2-CH_2Cl$ | O | N | $OCH_3$ | $CH_3$ | $-CH_2-CN$ | |
| 84 | $-CH_2-CH_2Cl$ | O | N | $OC_2H_5$ | $CH_3$ | $CH_3$ | |

TABELLE 1 (Fortsetzung)

| Nr. | A | X | E | $R_3$ | $R_4$ | $R_5$ | Smp. °C |
|---|---|---|---|---|---|---|---|
| 85 | $-CH_2-CH_2Cl$ | O | N | $-OCH_2-CF_3$ | $CH_3$ | $CH_3$ | |
| 86 | $-CH_2-CH_2Cl$ | O | N | $-OCH_2-CF_3$ | $CH_3$ | $OCH_3$ | |
| 87 | $-CH_2-CH_2Cl$ | O | N | $OC_2H_5$ | $CH_3$ | $OCH_3$ | |
| 88 | $-CH_2-CH_2Cl$ | O | CH | Cl | $CH_3$ | $CH_3$ | |
| 89 | $-CH_2-CH_2Cl$ | O | CH | $CH_3$ | $CH_3$ | $CH_3$ | |
| 90 | $-CH_2-CH_2Cl$ | O | CH | $CH_3$ | $CH_3$ | $OCH_3$ | |
| 91 | $-CH_2-C\equiv CH$ | O | CH | $OCH_3$ | n-$C_3H_7$ | $CH_3$ | |
| 92 | $-CH_2-C\equiv CH$ | O | CH | $OCH_3$ | $CH_3$ | $OCH_3$ | |
| 93 | $-CH_2-C\equiv CH$ | O | CH | $OCH_3$ | $C_2H_5$ | $CH_3$ | |
| 94 | $-CH_2-C\equiv CH$ | O | CH | $OCH_3$ | $CH_3$ | $-CH_2-CH_2-CN$ | |
| 95 | $-CH_2-C\equiv CH$ | O | CH | $OCH_3$ | $CH_3$ | H | |
| 96 | $-CH_2-C\equiv CH$ | O | CH | $OCH_3$ | $CH_3$ | $-CH_2-CN$ | |
| 97 | $-CH_2-C\equiv CH$ | O | CH | $OCH_3$ | $CH_3$ | $CH_3$ | |
| 98 | $-CH_2-C\equiv CH$ | S | CH | $OCH_3$ | $CH_3$ | $OCH_3$ | |
| 99 | $-CH_2-C\equiv CH$ | S | CH | $OCH_3$ | $CH_3$ | $CH_3$ | |
| 100 | $-CH_2-C\equiv CH$ | O | N | $OCH_3$ | $CH_3$ | $OCH_3$ | |
| 101 | $-CH_2-C\equiv CH$ | O | N | $OCH_3$ | $C_2H_5$ | $OCH_3$ | |
| 102 | $-CH_2-C\equiv CH$ | O | N | $OCH_3$ | $C_2H_5$ | $CH_3$ | |
| 103 | $-CH_2-C\equiv CH$ | O | N | $OCH_3$ | $CH_3$ | $-CH_2-CH_2-CN$ | |
| 104 | $-CH_2-C\equiv CH$ | O | N | $OCH_3$ | $CH_3$ | $-CH_2-CN$ | |
| 105 | $-CH_2-C\equiv CH$ | O | N | $OC_2H_5$ | $CH_3$ | $CH_3$ | |
| 106 | $-CH_2-C\equiv CH$ | O | N | $-OCH_2-CF_3$ | $CH_3$ | $CH_3$ | 160—164 |
| 107 | $-CH_2-C\equiv CH$ | O | N | $-OCH_2-CF_3$ | $CH_3$ | $OCH_3$ | |
| 108 | $-CH_2-C\equiv CH$ | O | N | $OC_2H_5$ | $CH_3$ | $OCH_3$ | |
| 109 | $-CH_2-C\equiv CH$ | O | CH | Cl | $CH_3$ | $CH_3$ | |
| 110 | $-CH_2-C\equiv CH$ | O | CH | $CH_3$ | $CH_3$ | $CH_3$ | |
| 111 | $-CH_2-C\equiv CH$ | O | CH | $CH_3$ | $CH_3$ | $OCH_3$ | |
| 112 | $-CH_2-CH=CH_2$ | O | CH | $OCH_3$ | n-$C_3H_7$ | $CH_3$ | |
| 113 | $-CH_2-CH=CH_2$ | O | CH | $OCH_3$ | $CH_3$ | $OCH_3$ | |
| 114 | $-CH_2-CH=CH_2$ | O | CH | $OCH_3$ | $C_2H_5$ | $CH_3$ | |

TABELLE 1 (Fortsetzung)

| Nr. | A | X | E | $R_3$ | $R_4$ | $R_5$ | Smp. °C |
|---|---|---|---|---|---|---|---|
| 115 | $-CH_2-CH=CH_2$ | O | CH | $OCH_3$ | $CH_3$ | $-CH_2-CH_2-CN$ | |
| 116 | $-CH_2-CH=CH_2$ | O | CH | $OCH_3$ | $CH_3$ | H | |
| 117 | $-CH_2-CH=CH_2$ | O | CH | $OCH_3$ | $CH_3$ | $-CH_2-CN$ | |
| 118 | $-CH_2-CH=CH_2$ | O | CH | $OCH_3$ | $CH_3$ | $CH_3$ | |
| 119 | $-CH_2-CH=CH_2$ | S | CH | $OCH_3$ | $CH_3$ | $OCH_3$ | |
| 120 | $-CH_2-CH=CH_2$ | S | CH | $OCH_3$ | $CH_3$ | $CH_3$ | |
| 121 | $-CH_2-CH=CH_2$ | O | N | $OCH_3$ | $CH_3$ | $OCH_3$ | |
| 122 | $-CH_2-CH=CH_2$ | O | N | $OCH_3$ | $C_2H_5$ | $OCH_3$ | |
| 123 | $-CH_2-CH=CH_2$ | O | N | $OCH_3$ | $C_2H_5$ | $CH_3$ | |
| 124 | $-CH_2-CH=CH_2$ | O | N | $OCH_3$ | $CH_3$ | $-CH_2-CH_2-CN$ | |
| 125 | $-CH_2-CH=CH_2$ | O | N | $OCH_3$ | $CH_3$ | $-CH_2-CN$ | |
| 126 | $-CH_2-CH=CH_2$ | O | N | $OC_2H_5$ | $CH_3$ | $CH_3$ | |
| 127 | $-CH_2-CH=CH_2$ | O | N | $-OCH_2-CF_3$ | $CH_3$ | $CH_3$ | |
| 128 | $-CH_2-CH=CH_2$ | O | N | $-OCH_2-CF_3$ | $CH_3$ | $OCH_3$ | |
| 129 | $-CH_2-CH=CH_2$ | O | N | $OC_2H_5$ | $CH_3$ | $OCH_3$ | |
| 130 | $-CH_2-CH=CH_2$ | O | CH | Cl | $CH_3$ | $CH_3$ | |
| 131 | $-CH_2-CH=CH_2$ | O | CH | $CH_3$ | $CH_3$ | $CH_3$ | |
| 132 | $-CH_2-CH=CH_2$ | O | CH | $CH_3$ | $CH_3$ | $OCH_3$ | |
| 133 | $-CF_2-CHF_2$ | O | CH | $OCH_3$ | n-$C_3H_7$ | $CH_3$ | |
| 134 | $-CF_2-CHF_2$ | O | CH | $OCH_3$ | $CH_3$ | $OCH_3$ | |
| 135 | $-CF_2-CHF_2$ | O | CH | $OCH_3$ | $C_2H_5$ | $CH_3$ | |
| 136 | $-CF_2-CHF_2$ | O | CH | $OCH_3$ | $CH_3$ | $-CH_2-CH_2-CN$ | |
| 137 | $-CF_2-CHF_2$ | O | CH | $OCH_3$ | $CH_3$ | H | |
| 138 | $-CF_2-CHF_2$ | O | CH | $OCH_3$ | $CH_3$ | $-CH_2-CN$ | |
| 139 | $-CF_2-CHF_2$ | O | CH | $OCH_3$ | $CH_3$ | $CH_3$ | |
| 140 | $-CF_2-CHF_2$ | S | CH | $OCH_3$ | $CH_3$ | $OCH_3$ | |
| 141 | $-CF_2-CHF_2$ | S | CH | $OCH_3$ | $CH_3$ | $CH_3$ | |
| 142 | $-CF_2-CHF_2$ | O | N | $OCH_3$ | $CH_3$ | $OCH_3$ | |
| 143 | $-CF_2-CHF_2$ | O | N | $OCH_3$ | $C_2H_5$ | $OCH_3$ | |
| 144 | $-CF_2-CHF_2$ | O | N | $OCH_3$ | $C_2H_5$ | $CH_3$ | |

TABELLE 1 (Fortsetzung)

| Nr. | A | X | E | $R_3$ | $R_4$ | $R_5$ | Smp. °C |
|---|---|---|---|---|---|---|---|
| 145 | $-CF_2-CHF_2$ | O | N | $OCH_3$ | $CH_3$ | $-CH_2-CH_2-CN$ | |
| 146 | $-CF_2-CHF_2$ | O | N | $OCH_3$ | $CH_3$ | $-CH_2-CN$ | |
| 147 | $-CF_2-CHF_2$ | O | N | $OC_2H_5$ | $CH_3$ | $CH_3$ | |
| 148 | $-CF_2-CHF_2$ | O | N | $-OCH_2-CF_3$ | $CH_3$ | $CH_3$ | |
| 149 | $-CF_2-CHF_2$ | O | N | $-OCH_2-CF_3$ | $CH_3$ | $OCH_3$ | |
| 150 | $-CF_2-CHF_2$ | O | N | $OC_2H_5$ | $CH_3$ | $OCH_3$ | |
| 151 | $-CF_2-CHF_2$ | O | CH | Cl | $CH_3$ | $CH_3$ | |
| 152 | $-CF_2-CHF_2$ | O | CH | $CH_3$ | $CH_3$ | $CH_3$ | |
| 153 | $-CF_2-CHF_2$ | O | CH | $CH_3$ | $CH_3$ | $OCH_3$ | |
| 154 | $CF_3$ | O | CH | $OCH_3$ | $CH_3$ | $CH_3$ | |
| 155 | $CF_3$ | O | CH | $OCH_3$ | $CH_3$ | $OCH_3$ | |
| 156 | $CF_3$ | O | N | $OCH_3$ | $CH_3$ | $OCH_3$ | |
| 157 | $CF_3$ | O | N | $OCH_3$ | $CH_3$ | $CH_3$ | 181—182 |
| 158 | $C_2F_5$ | O | CH | $OCH_3$ | $CH_3$ | $CH_3$ | 189—190 |
| 159 | $C_2F_5$ | O | CH | $OCH_3$ | $CH_3$ | $OCH_3$ | |
| 160 | $C_2F_5$ | O | N | $OCH_3$ | $CH_3$ | $CH_3$ | 175—176 |
| 161 | $C_2F_5$ | O | N | $OCH_3$ | $CH_3$ | $OCH_3$ | |
| 162 | $-CF_2-CHFCl$ | O | CH | $OCH_3$ | $CH_3$ | $CH_3$ | 163—166 |
| 163 | $-CF_2-CHFCl$ | O | N | $OCH_3$ | $CH_3$ | $CH_3$ | |
| 164 | $-CF_2-CHFCl$ | O | CH | $OCH_3$ | $CH_3$ | $OCH_3$ | |
| 165 | $-CF_2-CHFCl$ | O | N | $OCH_3$ | $CH_3$ | $OCH_3$ | |
| 166 | $-CH_2-OCH_3$ | O | CH | $OCH_3$ | $CH_3$ | $CH_3$ | |
| 167 | $-CH_2-OCH_3$ | O | CH | $OCH_3$ | $CH_3$ | $OCH_3$ | |
| 168 | $-CH_2-OCH_3$ | O | N | $OCH_3$ | $CH_3$ | $CH_3$ | |
| 169 | $-CH_2-OCH_3$ | O | N | $OCH_3$ | $CH_3$ | $OCH_3$ | |
| 170 | $-CH_2-C(CH_3)=CH_2$ | O | CH | $OCH_3$ | $CH_3$ | $OCH_3$ | |
| 171 | $-CH_2-C(CH_3)=CH_2$ | O | CH | $OCH_3$ | $CH_3$ | $CH_3$ | |
| 172 | $-CH_2-C(CH_3)=CH_2$ | O | N | $OCH_3$ | $CH_3$ | $CH_3$ | 153—154 |
| 173 | $-CH_2-C(CH_3)=CH_2$ | O | N | $OCH_3$ | $CH_3$ | $OCH_3$ | |
| 174 | $-CCl=CHCl$ | O | CH | $OCH_3$ | $CH_3$ | $CH_3$ | 233—234 |

TABELLE 1 (Fortsetzung)

| Nr. | A | X | E | $R_3$ | $R_4$ | $R_5$ | |
|---|---|---|---|---|---|---|---|
| 175 | $-CCl=CHCl$ | O | CH | $OCH_3$ | $CH_3$ | $OCH_3$ | |
| 176 | $-CCl=CHCl$ | O | N | $OCH_3$ | $CH_3$ | $CH_3$ | 206—207 |
| 177 | $-CCl=CHCl$ | O | N | $OCH_3$ | $CH_3$ | $OCH_3$ | |
| 178 | $CHF_2$ | O | N | $OCH_3$ | $CH_3$ | $CH_3$ | 171—172 |
| 179 | $CHF_2$ | O | N | $SCH_3$ | $CH_3$ | $CH_3$ | 184—185 |
| 180 | $-CF_2-CHFCl$ | O | CH | $CH_3$ | $CH_3$ | $CH_3$ | |
| 181 | $CHF_2$ | S | N | $SCH_3$ | $CH_3$ | $CH_3$ | |
| 182 | $CHF_2$ | O | N | $i\text{-}C_3H_7$ | $CH_3$ | $CH_3$ | 150—153 |
| 183 | $CHF_2$ | S | N | $i\text{-}OC_3H_7$ | $CH_3$ | $CH_3$ | 178—180 |
| 184 | $CHF_2$ | O | N | $OCH_3$ | H | H | >250 |
| 185 | $CHF_2$ | O | N | $OCH_3$ | H | $t\text{-}C_4H_9$ | 213—214 |
| 186 | $CHF_2$ | O | N | $OCH_3$ | H | $C_2H_5$ | 191—192 |
| 187 | $CHF_2$ | O | N | $OCH_3$ | H | $CH_3$ | 188—190 |
| 188 | $CHF_2$ | S | N | $OCH_3$ | $CH_3$ | $CH_3$ | 188—190 |
| 189 | $CHF_2$ | O | N | $OCH_3$ | $CH_3$ | $OCH_3$ | 122 (Zers. |
| 190 | $CHF_2$ | O | N | Cl | $CH_3$ | $CH_3$ | 199—200 |
| 191 | $-(CH_2)_2-OCH_3$ | O | N | $OCH_3$ | $CH_3$ | $CH_3$ | 173—174 |
| 192 | $-CH_2-CH_2Cl$ | O | N | $OCH_3$ | $CH_3$ | $CH_3$ | 181—182 |
| 193 | $-CF_2-CHF_2$ | O | N | $OCH_3$ | $CH_3$ | $CH_3$ | 169—170 |
| 194 | $-CH_2-C\equiv CH$ | O | N | $OCH_3$ | $CH_3$ | $CH_3$ | 197—199 |
| 195 | $-CH_2-CH=CH-CH_3$ | O | N | $OCH_3$ | $CH_3$ | $CH_3$ | 182—184 |
| 196 | $-CH_2-CH=CH_2$ | O | N | $OCH_3$ | $CH_3$ | $CH_3$ | 177—180 |
| 197 | $CHF_2$ | O | N | $OCH_3$ | $CH_3$ | $C_2H_5$ | 172—173 |
| 198 | $-(CH_2)_2-O-C_2H_5$ | O | N | $OCH_3$ | $CH_3$ | $CH_3$ | |
| 199 | $-(CH_2)_2-O-C_2H_5$ | O | N | $OCH_3$ | $CH_3$ | $OCH_3$ | |
| 200 | $-(CH_2)_2-O-C_2H_5$ | O | CH | $OCH_3$ | $CH_3$ | $CH_3$ | |
| 201 | $-(CH_2)_2-O-C_2H_5$ | O | CH | $OCH_3$ | $CH_3$ | $OCH_3$ | |
| 202 | $-(CH_2)_2-SCH_3$ | O | N | $OCH_3$ | $CH_3$ | $CH_3$ | |
| 203 | $-(CH_2)_2-SCH_3$ | O | N | $OCH_3$ | $CH_3$ | $OCH_3$ | |
| 204 | $-(CH_2)_2-SCH_3$ | O | CH | $OCH_3$ | $CH_3$ | $CH_3$ | |

TABELLE 1 (Fortsetzung)

| Nr. | A | X | E | $R_3$ | $R_4$ | $R_5$ | Smp. °C |
|---|---|---|---|---|---|---|---|
| 205 | $-(OH_2)_2-SCH_3$ | O | CH | $OCH_3$ | $CH_3$ | $OCH_3$ | |
| 206 | $-(CH_2)_2-OCH_3$ | O | N | $OCH_3$ | $CH_3$ | H | |
| 207 | $-(CH_2)_2-OCH_3$ | S | N | $OCH_3$ | $CH_3$ | $CH_3$ | |
| 208 | $-(CH_2)_2-OCH_3$ | S | N | $OCH_3$ | $CH_3$ | H | |
| 209 | $-(CH_2)_2-SCH_3$ | S | N | $OCH_3$ | $CH_3$ | $CH_3$ | |
| 210 | $-(CH_2)_2-SCH_3$ | S | N | $OCH_3$ | $CH_3$ | $OCH_3$ | |
| 211 | $-(CH_2)_2-SCH_3$ | S | CH | $OCH_3$ | $CH_3$ | $CH_3$ | |
| 212 | $-(CH_2)_2-SCH_3$ | S | CH | $OCH_3$ | $CH_3$ | $OCH_3$ | |
| 213 | $-(CH_2)_2-SCH_3$ | S | CH | $OCH_3$ | $CH_3$ | H | |
| 214 | $-(CH_2)_2-SCH_3$ | S | N | $OCH_3$ | $CH_3$ | H | |
| 215 | $-CCl=CHCl$ | O | N | $OCH_3$ | $CH_3$ | H | |
| 216 | $-CCl=CHCl$ | O | CH | $OCH_3$ | $CH_3$ | H | |
| 217 | $-CH_2-CH_2Cl$ | O | N | $OCH_3$ | $CH_3$ | H | |
| 218 | $-CH_2-C\equiv CH$ | O | N | $OCH_3$ | $CH_3$ | H | |
| 219 | $-CH_2-CH=CH_2$ | O | N | $OCH_3$ | $CH_3$ | H | |
| 220 | $-CF_2-CHF_2$ | O | N | $OCH_3$ | $CH_3$ | H | |
| 221 | $CF_3$ | O | N | $OCH_3$ | $CH_3$ | H | |
| 222 | $CF_3$ | O | CH | $OCH_3$ | $CH_3$ | H | |
| 223 | $-C_2F_5$ | O | N | $OCH_3$ | $CH_3$ | H | |
| 224 | $-C_2F_5$ | O | CH | $OCH_3$ | $CH_3$ | H | |
| 225 | $-CF_2-CHFCl$ | O | N | $OCH_3$ | $CH_3$ | H | |
| 226 | $-CF_2-CHFCl$ | O | CH | $OCH_3$ | $CH_3$ | H | |
| 227 | $-CH_2-OCH_3$ | O | N | $OCH_3$ | $CH_3$ | H | |
| 228 | $-CH_2-OCH_3$ | O | CH | $OCH_3$ | $CH_3$ | H | |
| 229 | $CHF_2$ | O | CH | $OCHF_2$ | $CH_3$ | $CH_3$ | 194—195 |
| 230 | $CHF_2$ | S | CH | $OCHF_2$ | $CH_3$ | $CH_3$ | |
| 231 | $-CF_2-CHF_2$ | O | CH | $OCHF_2$ | $CH_3$ | $CH_3$ | |
| 232 | $C_2F_5$ | O | CH | $OCHF_2$ | $CH_3$ | $CH_3$ | |
| 233 | $CF_3$ | O | CH | $OCHF_2$ | $CH_3$ | $CH_3$ | |
| 234 | $-(CH_2)_2-OCH_3$ | O | CH | $OCHF_2$ | $CH_3$ | $CH_3$ | |

TABELLE 1 (Fortsetzung)

| Nr. | A | X | E | $R_3$ | $R_4$ | $R_5$ | Smp. °C |
|---|---|---|---|---|---|---|---|
| 235 | $-(CH_2)_2-OC_2H_5$ | O | CH | $OCHF_2$ | $CH_3$ | $CH_3$ | |
| 236 | $-CH_2-CH_2Cl$ | O | CH | $OCHF_2$ | $CH_3$ | $CH_3$ | |
| 237 | $-CCl=CHCl$ | O | CH | $OCHF_2$ | $CH_3$ | $CH_3$ | |
| 238 | $-CH_2-CH=CH_2$ | O | CH | $OCHF_2$ | $CH_3$ | $CH_3$ | |
| 239 | $-CH_2-C\equiv CH$ | O | CH | $OCHF_2$ | $CH_3$ | $CH_3$ | |
| 240 | $-CH_2-OCH_3$ | O | CH | $OCHF_2$ | $CH_3$ | $CH_3$ | |
| 241 | $-(CH_2)_2-SCH_3$ | O | CH | $OCHF_2$ | $CH_3$ | $CH_3$ | |
| 242 | $-(CH_2)_2-OCH_3$ | S | CH | $OCHF_2$ | $CH_3$ | $CH_3$ | |
| 243 | $CHF_2$ | O | CH | $SCHF_2$ | $CH_3$ | $CH_3$ | |
| 244 | $CHF_2$ | O | CH | $OCHF_2$ | $CH_3$ | H | |
| 245 | $CF_3$ | O | CH | $OCHF_2$ | $CH_3$ | H | |
| 246 | $-(CH_2)_2-OCH_3$ | O | CH | $OCHF_2$ | $CH_3$ | H | |
| 247 | $-(CH_2)-C\equiv CH$ | O | N | $OCH_3$ | $CH_3$ | $CH_3$ | 197—199 |
| 248 | $-CH_2-CH=CH-CH_3$ | O | N | $OCH_3$ | $CH_3$ | $CH_3$ | 182—184 |
| 249 | $-CH_2-CH=CH_2$ | O | N | $OCH_3$ | $CH_3$ | $CH_3$ | 177—180 |
| 250 | $CHF_2$ | O | N | Cl | $CH_3$ | $CH_3$ | 199—200 |
| 251 | $-(CH_2)_2-OCH_3$ | O | N | $OCH_3$ | $CH_3$ | $CH_3$ | 173—174 |
| 252 | $-CH_2-CH_2Cl$ | O | N | $OCH_3$ | $CH_3$ | $CH_3$ | 181—182 |
| 253 | $-CF_2-CHF_2$ | O | N | $OCH_2$ | $CH_3$ | $CH_3$ | 169—170 |
| 254 | $CHF_2$ | O | N | $OCH_3$ | $CH_3$ | $-CH_2CH_3$ | 172—173 |
| 255 | $-CH_2-C\equiv CH$ | O | N | $-OCH_2-CF_3$ | $CH_3$ | $CH_3$ | 160—164 |
| 256 | $CHF_2$ | S | N | $OCH_3$ | $CH_3$ | H | 197—198 |
| 257 | $CHF_2$ | O | N | $OCHF_2$ | $CH_3$ | $CH_3$ | |

TABELLE 2

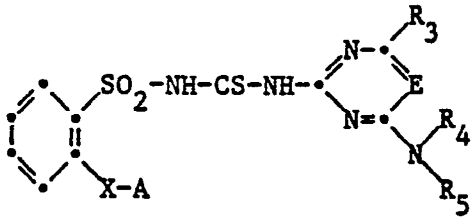

| Nr. | A | X | E | $R_3$ | $R_4$ | $R_5$ | Smp. °C |
|---|---|---|---|---|---|---|---|
| 301 | $CHF_2$ | O | CH | $OCH_3$ | $CH_3$ | $CH_3$ | 195—196 |
| 302 | $CHF_2$ | O | CH | $OCH_3$ | $CH_3$ | $OCH_3$ | |
| 303 | $CHF_2$ | O | N | $OCH_3$ | $CH_3$ | $CH_3$ | |
| 304 | $CHF_2$ | O | N | $OCH_3$ | $CH_3$ | $OCH_3$ | |
| 305 | $CHF_2$ | S | N | $OCH_3$ | $CH_3$ | $OCH_3$ | |
| 306 | $CHF_2$ | S | N | $OCH_3$ | $CH_3$ | $CH_3$ | |
| 307 | $CHF_2$ | S | CH | $OCH_3$ | $CH_3$ | $OCH_3$ | |
| 308 | $CHF_2$ | S | CH | $OCH_3$ | $CH_3$ | $CH_3$ | |
| 309 | $—CH_2—CH=CH_2$ | O | CH | $OCH_3$ | $CH_3$ | $CH_3$ | |
| 310 | $—CH_2—CH=CH_2$ | O | CH | $OCH_3$ | $CH_3$ | $OCH_3$ | |
| 311 | $—CH_2—CH=CH_2$ | O | N | $OCH_3$ | $CH_3$ | $CH_3$ | |
| 312 | $—CH_2—CH=CH_2$ | O | N | $OCH_3$ | $CH_3$ | $OCH_3$ | |
| 313 | $—(CH_2)_2—OCH_3$ | O | CH | $OCH_3$ | $CH_3$ | $OCH_3$ | |
| 314 | $—(CH_2)_2—OCH_3$ | O | CH | $OCH_3$ | $CH_3$ | $CH_3$ | |
| 315 | $—(CH_2)_2—OCH_3$ | O | N | $OCH_3$ | $CH_3$ | $OCH_3$ | |
| 316 | $—(CH_2)_2—OCH_3$ | O | N | $OCH_3$ | $CH_3$ | $CH_3$ | |

TABELLE 3

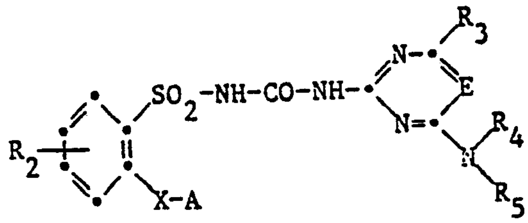

| Nr. | A | E | X | $R_2$ | $R_3$ | $R_4$ | $R_5$ | Smp. °C |
|---|---|---|---|---|---|---|---|---|
| 401 | $CHF_2$ | CH | O | 5-F | $OCH_3$ | $CH_3$ | $CH_3$ | |
| 402 | $CHF_2$ | CH | O | 5-F | $OCH_3$ | $CH_3$ | $OCH_3$ | |
| 403 | $CHF_2$ | N | O | 5-F | $OCH_3$ | $CH_3$ | $CH_3$ | 169—172 |
| 404 | $CHF_2$ | N | O | 5-F | $OCH_3$ | $CH_3$ | $OCH_3$ | |
| 405 | $CHF_2$ | CH | O | 5-Cl | $OCH_3$ | $CH_3$ | $CH_3$ | 210—211 |
| 406 | $CHF_2$ | CH | O | 5-Cl | $OCH_3$ | $CH_3$ | $OCH_3$ | |
| 407 | $CHF_2$ | N | O | 5-Cl | $OCH_3$ | $CH_3$ | $CH_3$ | 171—172 |
| 408 | $CHF_2$ | N | O | 5-Cl | $OCH_3$ | $CH_3$ | $OCH_3$ | |
| 409 | $CHF_2$ | CH | O | 6-Cl | $OCH_3$ | $CH_3$ | $CH_3$ | |
| 410 | $CHF_2$ | CH | O | 6-Cl | $OCH_3$ | $CH_3$ | $OCH_3$ | |
| 411 | $CHF_2$ | N | O | 6-Cl | $OCH_3$ | $CH_3$ | $CH_3$ | 149—151 |
| 412 | $CHF_2$ | N | O | 6-Cl | $OCH_3$ | $CH_3$ | $OCH_3$ | |
| 413 | $CHF_2$ | CH | O | 5-$CHF_2$ | $OCH_3$ | $CH_3$ | $CH_3$ | |
| 414 | $CHF_2$ | CH | O | 5-$CHF_2$ | $OCH_3$ | $CH_3$ | $OCH_3$ | |
| 415 | $CHF_2$ | N | O | 5-$CHF_2$ | $OCH_3$ | $CH_3$ | $CH_3$ | |
| 416 | $CHF_2$ | N | O | 5-$CHF_2$ | $OCH_3$ | $CH_3$ | $OCH_3$ | |
| 417 | $CHF_2$ | CH | O | 5-$COOCH_3$ | $OCH_3$ | $CH_3$ | $CH_3$ | |
| 418 | $CHF_2$ | CH | O | 5-$COOCH_3$ | $OCH_3$ | $CH_3$ | $OCH_3$ | |
| 419 | $CHF_2$ | N | O | 5-$COOCH_3$ | $OCH_3$ | $CH_3$ | $CH_3$ | |
| 420 | $CHF_2$ | N | O | 5-$COOCH_3$ | $OCH_3$ | $CH_3$ | $OCH_3$ | |
| 421 | $CHF_2$ | CH | O | 6-F | $OCH_3$ | $CH_3$ | $CH_3$ | |
| 422 | $CHF_2$ | CH | O | 6-F | $OCH_3$ | $CH_3$ | $OCH_3$ | |
| 423 | $CHF_2$ | N | O | 6-F | $OCH_3$ | $CH_3$ | $CH_3$ | |
| 424 | $CHF_2$ | N | O | 6-F | $OCH_3$ | $CH_3$ | $OCH_3$ | |
| 425 | $—CH_2—C{\equiv}CH$ | N | O | 5-Cl | $OCH_3$ | $CH_3$ | $CH_3$ | 194 (Zers.) |

TABELLE 3 (Fortsetzung)

| Nr. | A | E | X | $R_2$ | $R_3$ | $R_4$ | $R_5$ | Smp. °C |
|---|---|---|---|---|---|---|---|---|
| 426 | $—CH_2—CH—CH_2$ | N | O | 5-$CH_3$ | $OCH_3$ | $CH_3$ | $CH_3$ | 194—196 |
| 427 | $—CH_2—CH=CH_2$ | N | O | 5-F | $OCH_3$ | $CH_3$ | $CH_3$ | 192—193 |
| 428 | $—CH_2—C{\equiv}CH$ | N | O | 5-$CH_3$ | $OCH_3$ | $CH_3$ | $CH_3$ | 208—212 |
| 429 | $—CH_2—CH=CH_2$ | N | O | 5-F | $OCH_3$ | $CH_3$ | $CH_3$ | 192—193 |
| 430 | $—CH_2—C{\equiv}CH$ | N | O | 5-Cl | $OCH_3$ | $CH_3$ | $CH_3$ | 194—198 (Zers.) |
| 431 | $—CH_2—C{\equiv}CH$ | N | O | 5-$CH_3$ | $OCH_3$ | $CH_3$ | $CH_3$ | 208—212 |
| 432 | $—CH_2—C{\equiv}CH$ | N | O | 5-F | $OCH_3$ | $CH_3$ | $CH_3$ | 186—189 |
| 433 | $—CH_2—C(CH_3)=CH_2$ | N | O | 5-F | $OCH_3$ | $CH_3$ | $CH_3$ | 187—188 |
| 434 | $—CH_2—CH=CH_2$ | N | O | 5-Cl | $OCH_3$ | $CH_3$ | $CH_3$ | 174—176 |
| 435 | $—CH_2—C(CH_3)=CH_2$ | N | O | 5-Cl | $OCH_3$ | $CH_3$ | $CH_3$ | 153—155 (Zers.) |
| 436 | $—CH_2—CH=CH_2$ | N | O | 5-$CH_3$ | $OCH_3$ | $CH_3$ | $CH_3$ | 194—196 |

Formulierungsbeispiele

Beispiel 3

Formulierungsbeispiele für Wirkstoffe der Formel I (% = Gewichtsprozent)

| a) *Spritzpulver* | a) | b) | c) |
|---|---|---|---|
| Wirkstoff | 20% | 60% | 0,5% |
| Na-Ligninsulfonat | 5% | 5% | 5% |
| Na-Laurylsulfat | 3% | — | — |
| Na-Diisobutylnaphthalinsulfonat | — | 6% | 6% |
| Octylphenolpolyäthylenglykoläther (7—8 Mol AeO) | — | 2% | 2% |
| Hochdisperse Kieselsäure | 5% | 27% | 27% |
| Kaolin | 67% | — | — |
| Natriumchlorid | — | — | 59,5% |

Der wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) *Emulsion-Konzentrat*

| | a) | b) |
|---|---|---|
| Wirkstoff | 10% | 1% |
| Octylphenolpolyäthylenglykoläther (4—5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

c) *Stäubemittel*

| | a) | b) |
|---|---|---|
| Wirkstoff | 0,1% | 1% |
| Talkum | 99,9% | — |
| Kaolin | — | 99% |

Man erhält anwendungsfertige Stäubemittel, indem der Wirkstoff mit dem Träger vermischt und auf einer geeigneten Mühle vermahlen wird.

d) *Extruder Granulat*

| | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

e) *Umhüllungs-Granulat*

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) *Suspensions-Konzentrat*

| | a) | b) |
|---|---|---|
| Wirkstoff | 40% | 5% |
| Aethylenglykol | 10% | 10% |
| Nonylphenolpolyäthylenglykoläther (15 Mol AwO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) *Salzlösung*

| | |
|---|---|
| Wirkstoff | 5% |
| Isopropylamin | 1% |
| Octylphenolpolyäthylenglykoläther (78 Mol AeO) | 3% |
| Wasser | 91% |

Biologische Beispiele

Beispiel 4

Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70% gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5% eines handelsüblichen Flüssigdüngers (®Greenzit) zugesetzt. 12 Tage nach der Aussaat wird der Versuch ausgewertet und die Wirkung auf die Versuchspflanzen nach dem folgenden Masstab bewertet:

1: Pflanze nicht gekeimt oder total abgestorben
2—3: sehr starke Wirkung
4—6: mittlere Wirkung
7—8: schwache Wirkung
9: keine Wirkung (wie unbehandelte Kontrolle).

*pre-emergente Wirkung*:
Konzentration der Wirkstoffemulsion: 70,8 ppm

| Testpflanze / Wirkstoff Nr. | Nasturtium | Stellaria | Agrostis | Digitaria |
|---|---|---|---|---|
| 1 | 2 | — | — | — |
| 6 | 2 | 2 | 1 | — |
| 13 | 3 | 3 | 1 | 2 |
| 17 | 2 | — | — | — |
| 20 | 1 | 1 | 1 | — |
| 22 | 3 | — | — | — |
| 30 | 1 | 2 | 1 | 1 |
| 55 | 1 | 3 | 1 | 1 |
| 106 | 1 | 3 | 1 | 2 |
| 157 | 1 | 2 | 1 | 2 |
| 158 | 2 | 3 | 1 | 3 |
| 160 | 2 | 2 | 1 | — |
| 162 | 3 | — | — | — |
| 172 | 1 | 1 | 1 | — |
| 174 | 2 | 5 | 1 | — |
| 176 | 2 | 2 | 1 | — |
| 179 | 2 | — | — | — |
| 182 | 6 | — | — | — |
| 184 | 2 | — | — | — |
| 185 | 5 | — | — | — |
| 186 | 2 | — | — | — |
| 187 | 3 | — | — | — |
| 188 | 2 | — | — | — |
| 189 | 2 | — | — | — |
| 191 | 1 | 1 | 1 | 1 |
| 201 | 1 | 2 | 1 | 1 |
| 247 | 1 | 1 | 1 | — |
| 248 | 1 | 2 | 1 | — |

| Testpflanze / Wirkstoff Nr. | Nasturtium | Stellaria | Agrostis | Digitaria |
|---|---|---|---|---|
| 249 | 1 | 2 | 1 | 2 |
| 252 | 1 | 1 | 1 | 1 |
| 253 | 1 | 1 | 1 | 2 |
| 254 | 2 | — | — | — |
| 256 | 2 | 2 | 2 | 2 |
| 257 | 1 | 1 | 1 | 1 |
| 305 | 1 | 1 | 1 | 1 |
| 307 | 1 | 1 | 1 | 1 |
| 403 | 1 | 2 | 1 | 2 |
| 425 | 1 | 1 | 1 | 1 |
| 427 | 1 | 1 | 1 | 1 |
| 428 | 1 | 2 | 1 | 1 |
| 432 | 2 | 2 | 2 | 2 |
| 433 | 2 | 3 | 1 | 3 |
| 434 | 1 | 3 | 1 | 4 |
| 435 | 2 | 4 | 2 | 6 |
| 436 | 1 | 3 | 1 | 2 |

—: nicht geprüft.

Beispiel 5

Herbizidwirkung nach dem Auflaufen der Pflanzen (Kontaktwirkung)

Eine Anzahl Unkräuter und Kulturpflanzen, sowohl monokotyle wie dikotyle, wurden nach dem Auflaufen, im 4- bis 6-Blattstadium mit einer wässrigen Wirkstoffdispersion von 4 kg AS/ha gespritzt und dann bei 24° bis 26°C und 45—60% relativer Luftfeuchtigkeit gehalten. 15 Tage nach der Behandlung wird der Versuch ausgewertet und nach dem gleichen Massstab wie im pre-emergenten Versuch bewertet.

*post-emergente Wirkung*

Aufwandmenge: 4 kg Wirksubstanz/Hektar

| Verb. Nr. | Avena | Setaria | Lolium | Solanum | Sinapis | Stellaria | Phaseolus |
|---|---|---|---|---|---|---|---|
| 179 | 6 | 5 | 4 | 2 | 2 | 3 | 3 |
| 182 | 9 | 9 | 5 | 2 | 2 | 3 | 3 |
| 189 | 2 | 3 | 2 | 2 | 2 | 2 | 3 |

Beispiel 6

Wuchshemmung bei tropischen Bodendecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70% relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20% des Neuzuwachses bei unbehandelten Kontrollpflanzen) ohne dass dabei die Versuchspflanzen geschädigt wurden.

Beispiel 7

Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5—6 Trifolia-Blattstadium. Zu diesem Zeitpunkt werden die Pflanzen mit der wässrigen Brühe eines Wirkstoffes der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten am Haupttrieb.

Beispiel 8

Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zur unbehandelten Kontrolle eine Verringerung des Neuzuwachses (60—90% der Kontrolle), sowie teilweise eine Zunahme der Stengeldurchmesser auf.

Beispiel 9

Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10—30% im Vergleich zur unbehandelten Kontrolle.

**Patentansprüche für die Vertragsstaaten: BE CH DE FR GB IT LI NL**

1. N-Phenylsulfonyl-N'-pyrimidinyl- und -triazinyl-harnstoffe der allgemeinen Formel

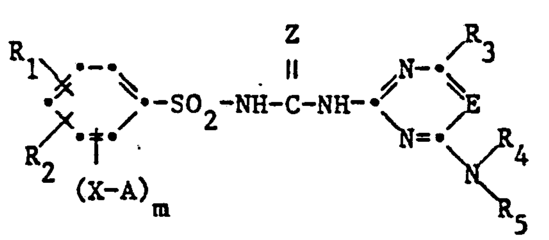

(I),

worin

A einen $C_3$—$C_6$-Alkinylrest, einen durch Halogen, $C_1$—$C_4$-Alkoxy, $C_1$—$C_4$-Alkylthio, $C_1$—$C_4$-Alkylsulfinyl, $C_1$—$C_4$-Alkylsulfonyl, $C_1$—$C_4$-Halogenalkoxy, $C_1$—$C_4$-Halogenalkylthio, $C_1$—$C_4$-Halogenalkylsulfinyl oder $C_1$—$C_4$-Halogenalkylsulfonyl substituierten $C_1$—$C_6$-Alkylrest oder einen durch die aufgezählten Reste substituierten $C_2$—$C_6$-Alkenylrest,

E die Methingruppe oder Stickstoff,

X Sauerstoff, Schwefel, eine Sulfinyl- oder Sulfonylbrücke,
Z Sauerstoff oder Schwefel,
m die Zahl eins oder zwei,
$R_1$ Wasserstoff, Halogen, $C_1$—$C_5$-Alkyl, $C_2$—$C_5$-Alkenyl oder einen Rest —Y—$R_6$,
$R_2$ Wasserstoff, Halogen, $C_1$—$C_5$-Alkyl, $C_2$—$C_5$-Alkenyl, $C_1$—$C_4$-Halogenalkyl, oder einen Rest —Y—$R_6$, —$COOR_7$, —$NO_2$ oder —CO—$NR_8$—$R_9$,
$R_3$ Wasserstoff, $C_1$—$C_4$-Alkyl, $C_1$—$C_4$-Alkoxy, $C_1$—$C_4$-Alkylthio, $C_1$—$C_4$-Halogenalkyl, $C_1$—$C_4$-Halogenalkoxy, Halogen oder Alkoxyalkyl mit höchstens 4 Kohlenstoffatomen,
$R_4$ Wasserstoff, Methyl oder Aethyl,
$R_5$ Wasserstoff, $C_1$—$C_4$-Alkyl, $C_1$—$C_3$-Alkoxy, Methoxymethyl, Cyanomethyl oder Cyanoäthyl,
$R_6$ und $R_7$ je $C_1$—$C_5$-Alkyl, $C_2$—$C_5$-Alkenyl oder $C_2$—$C_6$-Alkinyl,
$R_8$ und $R_9$ unabhängig voneinander Wasserstoff, $C_1$—$C_5$-Alkyl, $C_2$—$C_5$-Alkenyl oder $C_2$—$C_6$-Alkinyl und
Y Sauerstoff, Schwefel, eine Sulfinyl- oder Sulfonylbrücke bedeuten, wobei A auch $C_2$—$C_6$-Alkenyl bedeuten kann, wenn X für Sauerstoff steht, sowie die Salze dieser Verbindungen.

2. Verbindungen gemäss Anspruch 1, dadurch gekennzeichnet, dass Z Sauerstoff bedeutet.

3. Verbindungen gemäss Anspruch 1, dadurch gekennzeichnet, dass m die Zahl eins bedeutet.

4. Verbindungen gemäss Anspruch 1, dadurch gekennzeichnet, dass ein Rest —X—A im Phenylring die 2-Stellung zur Sulfonamidgruppe besetzt.

5. Verbindungen gemäss Anspruch 1, dadurch gekennzeichnet, dass die Reste $R_4$ und $R_5$ zusammen höchstens 4 Kohlenstoffatome enthalten.

6. Verbindungen gemäss Anspruch 1, dadurch gekennzeichnet, dass X Sauerstoff bedeutet.

7. Verbindungen gemäss Anspruch 1, dadurch gekennzeichnet, dass X und Z Sauerstoff und m die Zahl eins bedeuten, $R_4$ und $R_5$ zusammen nicht mehr als 4 Kohlenstoffatome enthalten und der Rest —X—A im Phenylring die 2-Stellung zur Sulfonamidgruppe besetzt.

8. Verbindungen gemäss Anspruch 7, dadurch gekennzeichnet, dass A für $C_1$—$C_4$-Halogenalkyl steht.

9. Verbindungen gemäss Anspruch 7, dadurch gekennzeichnet, dass A $C_3$—$C_4$-Alkinyl, $C_2$—$C_6$-Alkenyl oder -Halogenalkenyl oder durch $C_1$—$C_4$-Alkoxy, $C_1$—$C_4$-Alkylthio, $C_1$—$C_4$-Alkylsulfinyl oder $C_1$—$C_4$-Alkylsulfonyl substituiertes $C_1$—$C_3$-Alkyl bedeutet.

10. Verbindungen gemäss einem der Ansprüche 8 oder 9, dadurch gekennzeichnet, dass $R_1$ Wasserstoff und $R_2$ Wasserstoff, Halogen, Methyl oder —COO—$C_1$—$C_3$-Alkyl bedeuten.

11. Verbindungen gemäss Anspruch 10, dadurch gekennzeichnet, dass $R_1$ und $R_2$ Wasserstoff bedeuten.

12. Verbindungen gemäss Anspruch 11, dadurch gekennzeichnet, dass $R_3$ Methoxy, Aethoxy, Difluormethoxy, 2,2,2-Trifluoräthoxy, Methyl, Aethyl, Fluormethyl oder Chlormethyl, $R_4$ Wasserstoff, Methyl oder Aethyl und $R_5$ Methyl oder Aethyl bedeuten.

13. N-(2-Difluormethoxyphenyl-sulfonyl)-N'-(4-dimethylamino-6-methoxy-1,3,5-triazin-2-yl)-harnstoff gemäss Anspruch 1.

14. N-(2-Difluormethoxyphenyl-sulfonyl)-N'-(4-dimethylamino-6-methoxypyrimidin-2-yl)-harnstoff gemäss Anspruch 1.

15. N-(2-Difluormethoxyphenyl-sulfonyl)-N'-(4-methylamino-6-methoxypyrimidin-2-yl)-harnstoff gemäss Anspruch 1.

16. Verfahren zur Herstellung der Verbindungen der Formel I, Anspruch 1, dadurch gekennzeichnet, dass man ein Phenylsulfonamid der Formel II

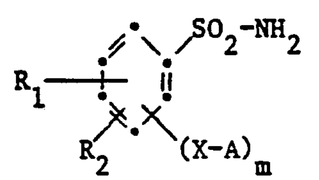

(II),

worin A, $R_1$, $R_2$, X und m die unter Formel I gegebene Bedeutung haben, in Gegenwart einer Base mit einem N-Pyrimidinyl- oder -Triazinylcarbamat der Formel

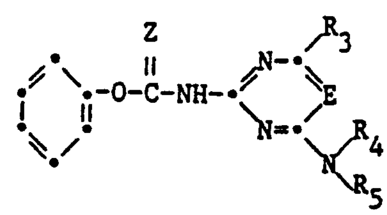

(III),

worin E, $R_3$, $R_4$, $R_5$ und Z die unter Formel I gegebene Bedeutung haben, umsetzt und gegebenenfalls in ihre Salze überführt.

17. Verfahren zur Herstellung der Verbindungen der Formel I, Anspruch 1, dadurch gekennzeichnet, dass man ein Phenylsulfonylisocyanat oder -isothiocyanat der Formel IV

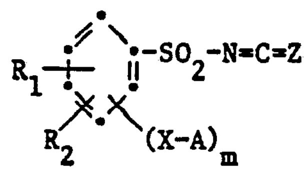

(IV),

worin A, $R_1$, $R_2$, m, X und Z die unter Formel I gegebene Bedeutung haben, gegebenenfalls in Gegenwart einer Base, mit einem Amin der Formel V

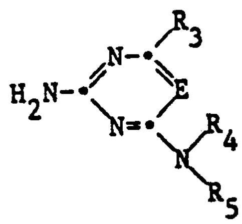

(V),

worin E, $R_3$, $R_4$ und $R_5$ die unter Formel I gegebene Bedeutung haben, umsetzt und gegebenenfalls in ihre Salze überführt.

18. Verfahren zur Herstellung der Verbindung der Formel I, Anspruch 1, dadurch gekennzeichnet, dass man Sulfonamide der in Anspruch 16 angegebenen Formel II gegebenenfalls in Gegenwart einer Base mit einem Isocyanat oder Isothiocyanat der Formel VI

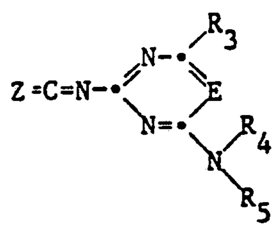

(VI),

worin E, $R_3$, $R_4$, $R_5$ und Z die unter Formel I gegebene Bedeutungen haben, umsetzt und gegebenenfalls in ihre Salze überführt.

19. Verfahren zur Herstellung der Verbindungen der Formel I, Anspruch 1, dadurch gekennzeichnet, dass man ein N-Phenylsulfonylcarbamat der Formel VII

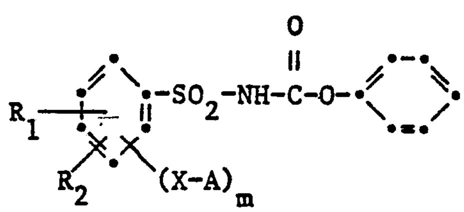

(VII),

worin A, $R_1$, $R_2$, m und X die unter Formel I gegebene Bedeutung haben, mit einem Amin der in Anspruch 17 angegebenen Formel V umsetzt und gegebenenfalls in ihre Salze überführt.

20. Verfahren zur Herstellung der Verbindungen der Formel I, Anspruch 1, dadurch gekennzeichnet, dass man einen Sulfonylharnstoff der Formel VIII

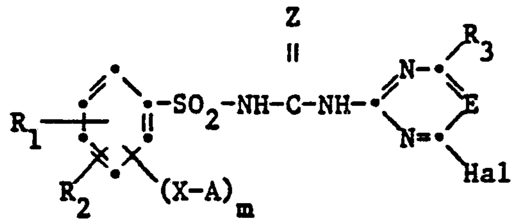

(VIII),

worin A, E, $R_1$, $R_2$, $R_3$, X, Z und m die unter Formel I gegebene Bedeutung haben und Hal für Chlor oder Brom steht, mit einem Amin der Formel IX

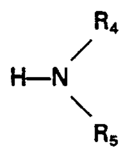

(IX),

worin $R_4$ und $R_5$ die unter Formel I angegebene Bedeutung haben, umsetzt und gegebenenfalls in ihre Salze überführt.

21. Verfahren zur Herstellung von Additionssalzen der Formel I gemäss einem der Ansprüche 16 bis 20, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

22. Ein herbizides und den Pflanzenwuchs regulierendes Mittel, dadurch gekennzeichnet, dass es neben Träger- und/oder anderen Zuschlagstoffen als Wirkstoff mindestens einen N-Phenylsulfonyl-N'-triazinyl- oder pyrimidinyl-harnstoff der Formel I, Anspruch 1, enthält.

23. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

24. Die Verwendung der N-Phenylsulfonyl-N'-triazinyl- oder -pyrimidinyl-harnstoffe der Formel I, Anspruch 1, oder sie enthaltender Mittel zur Regulierung des Pflanzenwachstums.

25. Die Verwendung der N-Phenylsulfonyl-N'-triazinyl- oder -pyrimidinyl-harnstoffe der Formel I, oder sie enthaltender Mittel, gemäss Anspruch 23, sur selektiven pre- oder postemergenten Bekämpfung von Unkräutern in Nutzpflanzenkulturen.

26. Die Verwendung der N-Phenylsulfonyl-N'-triazinyl- oder -pyrimidinyl-harnstoffe der Formel I, oder sie enthaltender Mittel, gemäss Anspruch 24, zur Unterdrückung des Pflanzenwachstums über das 2-Blattstadium hinaus, dadurch gekennzeichnet, dass die Wirkstoffe preemergent angewendet werden.

27. Die Verwendung gemäss Anspruch 25 in Zuckerrohr-, Getreide-, Mais-, Reis- und Baumwollkulturen.

28. Die Verwendung gemäss Anspruch 25 in Sojakulturen.

29. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

30. Die Verwendung gemäss Anspruch 29 in Sojakulturen.

31. Die Verwendung gemäss Anspruch 24 bei Bodendecker-Leguminosen.

**Patentansprüche für den Vertragsstaat: AT**

1. Ein herbizides und den Pflanzenwuchs regulierendes Mittel, dadurch gekennzeichnet, dass es neben Träger- und/oder anderen Zuschlagstoffen als Wirkstoff mindestens einen N-Phenylsulfonyl-N'-triazinyl- oder -pyrimidinyl-harnstoff der Formel I

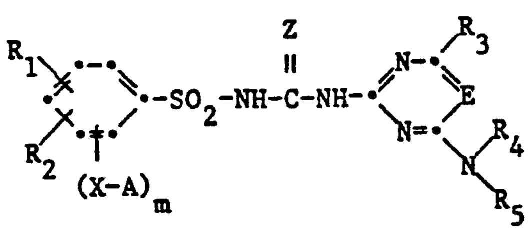

(I),

oder ein Salz davon enthält, worin

A einen $C_3$—$C_6$-Alkinylrest, einen durch Halogen, $C_1$—$C_4$-Alkoxy, $C_1$—$C_4$-Alkylthio, $C_1$—$C_4$-Alkylsulfinyl,

$C_1$—$C_4$-Alkylsulfonyl, $C_1$—$C_4$-Halogenalkoxy, $C_1$—$C_4$-Halogenalkylthio, $C_1$—$C_4$-Halogenalkylsulfinyl oder $C_1$—$C_4$-Halogenalkylsulfonyl substituierten $C_1$—$C_6$-Alkylrest oder einen durch die aufgezählten Reste substituierten $C_2$—$C_6$-Alkenylrest,

E die Methingruppe oder Stickstoff,

X Sauerstoff, Schwefel, eine Sulfinyl- oder Sulfonylbrücke,

Z Sauerstoff oder Schwefel,

m die Zahl eins oder zwei,

$R_1$ Wasserstoff, Halogen, $C_1$—$C_5$-Alkyl, $C_2$—$C_5$-Alkenyl oder einen Rest —Y—$R_6$,

$R_2$ Wasserstoff, Halogen, $C_1$—$C_5$-Alkyl, $C_2$—$C_5$-Alkenyl, $C_1$—$C_4$-Halogenalkyl, oder einen Rest —Y—$R_6$, —$COOR_7$, —$NO_2$ oder —CO—$NR_8$—$R_9$,

$R_3$ Wasserstoff, $C_1$—$C_4$-Alkyl, $C_1$—$C_4$-Alkoxy, $C_1$—$C_4$-Alkylthio, $C_1$—$C_4$-Halogenalkyl, $C_1$—$C_4$-Halogenalkoxy, Halogen oder Alkoxyalkyl mit höchstens 4 Kohlenstoffatomen,

$R_4$ Wasserstoff, Methyl oder Aethyl,

$R_5$ Wasserstoff, $C_1$—$C_4$-Alkyl, $C_1$—$C_3$-Alkoxy, Methoxymethyl, Cyanomethyl oder Cyanoäthyl,

$R_6$ und $R_7$ je $C_1$—$C_5$-Alkyl, $C_2$—$C_5$-Alkenyl oder $C_2$—$C_6$-Alkinyl,

$R_8$ und $R_9$ unabhängig voneinander Wasserstoff, $C_1$—$C_5$-Alkyl, $C_2$—$C_5$-Alkenyl oder $C_2$—$C_6$-Alkinyl und

Y Sauerstoff, Schwefel, eine Sulfinyl- oder Sulfonylbrücke bedeuten, wobei A auch $C_2$—$C_6$-Alkenyl bedeuten kann, wenn X für Sauerstoff steht, sowie die Salze dieser Verbindungen.

2. Mittel gemäss Anspruch 1, dadurch gekennzeichnet, dass Z Sauerstoff bedeutet.

3. Mittel gemäss Anspruch 1, dadurch gekennzeichnet, dass m die Zahl eins bedeutet.

4. Mittel gemäss Anspruch 1, dadurch gekennzeichnet, dass ein Rest —X—A im Phenylring die 2-Stellung zur Sulfonamidgruppe besetzt.

5. Mittel gemäss Anspruch 1, dadurch gekennzeichnet, dass die Reste $R_4$ und $R_5$ zusammen höchstens 4 Kohlenstoffatome enthalten.

6. Mittel gemäss Anspruch 1, dadurch gekennzeichnet, dass X Sauerstoff bedeutet.

7. Mittel gemäss Anspruch 1, dadurch gekennzeichnet, dass X und Z Sauerstoff und mi die Zahl eins bedeuten, $R_4$ und $R_5$ zusammen nicht mehr als 4 Kohlenstoffatome enthalten und der Rest —X—A im Phenylring die 2-Stellung zur Sulfonamidgruppe besetzt.

8. Mittel gemäss Anspruch 7, dadurch gekennzeichnet, dass A für $C_1$—$C_4$-Halogenalkyl steht.

9. Mittel gemäss Anspruch 7, dadurch gekennzeichnet, dass A $C_3$—$C_4$-Alkinyl, $C_2$—$C_6$-Alkenyl oder -Halogenalkenyl oder durch $C_1$—$C_4$-Alkoxy, $C_1$—$C_4$-Alkylthio, $C_1$—$C_4$-Alkylsulfinyl oder $C_1$—$C_4$-Alkylsulfonyl substituiertes $C_1$—$C_3$-Alkyl bedeutet.

10. Mittel gemäss einem der Ansprüche 8 oder 9, dadurch gekennzeichnet, dass $R_1$ Wasserstoff und $R_2$ Wasserstoff, Halogen, Methyl oder —COO—$C_1$—$C_3$-Alkyl bedeuten.

11. Mittel gemäss Anspruch 10, dadurch gekennzeichnet, dass $R_1$ und $R_2$ Wasserstoff bedeuten.

12. Mittel gemäss Anspruch 11, dadurch gekennzeichnet, dass $R_3$ Methoxy, Aethoxy, Difluormethoxy, 2,2,2-Trifluoräthoxy, Methyl, Aethyl, Fluormethyl oder Chlormethyl, $R_4$ Wasserstoff, Methyl oder Aethyl und $R_5$ Methyl oder Aethyl bedeuten.

13. Mittel gemäss Anspruch 1, dadurch gekennzeichnet, dass es als Wirkstoff N-(2-Difluormethoxyphenyl-sulfonyl)-N'-(4-dimethylamino-6-methoxy-1,3,5-triazin-2-yl)-harnstoff enthält.

14. Mittel gemäss Anspruch 1, dadurch gekennzeichnet, dass es als Wirkstoff N-(2-Difluormethoxyphenyl-sulfonyl)-N'-(4-dimethylamino-6-methoxy-pyrimidin-2-yl)-harnstoff enthält.

15. Mittel gemäss Anspruch 1, dadurch gekennzeichnet, dass es als Wirkstoff N-(2-Difluormethoxyphenyl-sulfonyl)-N'-(4-methylamino-6-methoxy-pyrimidin-2-yl)-harnstoff enthält.

16. Verfahren zur Herstellung der Verbindungen der Formel I, Anspruch 1, dadurch gekennzeichnet, dass man entweder

a) ein Phenylsulfonamid der Formel II

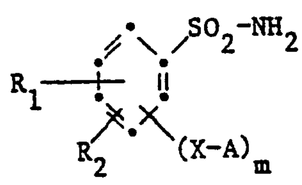

(II),

worin A, $R_1$, $R_2$, X und m die unter Formel I gegebene Bedeutung haben, in Gegenwart einer Base mit einem N-Pyrimidinyl- oder -Triazinylcarbamat der Formel

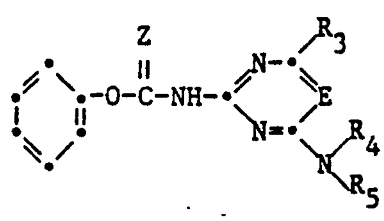

(III),

worin E, $R_3$, $R_4$, $R_5$ und Z die unter Formel I gegebene Bedeutung haben oder

b) ein Phenylsulfonylisocyanat oder -isothiocyanat der Formel IV

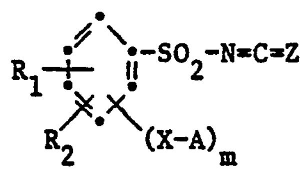

(IV),

worin A, $R_1$, $R_2$, m, X und Z die unter Formel I gegebene Bedeutung haben, gegebenenfalls in Gegenwart einer Base, mit einem Amin der Formel V

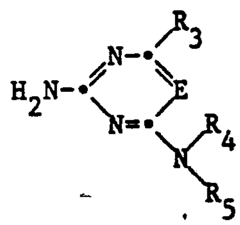

(V),

worin E, $R_3$, $R_4$ und $R_5$ die unter Formel I gegebene Bedeutung haben oder

c) Sulfonamide der oben angegebenen Formel II gegebenenfalls in Gegenwart einer Base mit einem Isocyanat oder Isothiocyanat der Formel VI

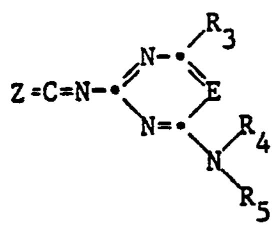

(VI),

worin E, $R_3$, $R_4$, $R_5$ und Z die unter Formel I gegebenen Bedeutungen haben oder

d) ein N-Phenylsulfonylcarbamat der Formel VII

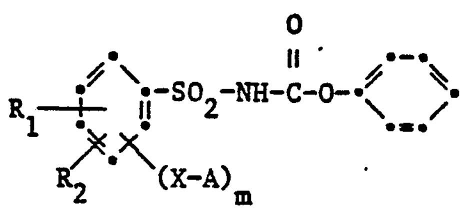

(VII),

worin A, $R_1$, $R_2$, m und X die unter Formel I gegebene Bedeutung haben, mit einem Amin der oben angegebenen Formel V oder

e) einen Sulfonylharnstoff der Formel VIII

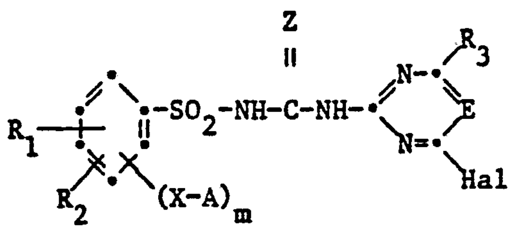

(VIII),

worin A, E, $R_1$, $R_2$, $R_3$, X, Z und m die unter Formel I gegebene Bedeutung haben und Hal für Chlor oder Brom steht, mit einem Amin der Formel IX

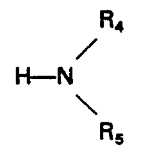

(IX),

worin $R_4$ und $R_5$ die unter Formel I angegebene Bedeutung haben, umsetzt und gegebenenfalls in ihre Salze überführt indem man einen Sulfonylharnstoff der Formel I mit einem Amin, einem Alkalimetall- oder Erdalkalimetallhydroxid oder einer quaternären Ammoniumbase umsetzt.

17. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

18. Die Verwendung der N-Phenylsulfonyl-N'-triazinyl- oder -pyrimidinyl-harnstoffe der Formel I, Anspruch 1, oder sie enthaltender Mittel zur Regulierung des Pflanzenwachstums.

19. Die Verwendung der N-Phenylsulfonyl-N'-triazinyl- oder -pyrimidinyl-harnstoffe der Formel I, oder sie enthaltender Mittel, gemäss Anspruch 17, zur selektiven pre- oder postemergenten Bekämpfung von Unkräutern in Nutzpflanzenkulturen.

20. Die Verwendung der N-Phenylsulfonyl-N'-triazinyl- oder -pyrimidinyl-harnstoffe der Formel I, oder sie enthaltender Mittel, gemäss Anspruch 18, zur Unterdrückung des Pflanzenwachstums über das 2-Blattstadium hinaus, dadurch gekennzeichnet, dass die Wirkstoffe preemergent angewendet werden.

21. Die Verwendung gemäss Anspruch 19 in Zuckerrohr-, Getreide-, Mais-, Reis- und Baumwollkulturen.

22. Die Verwendung gemäss Anspruch 19 in Sojakulturen.

23. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

24. Die Verwendung gemäss Anspruch 23 in Sojakulturen.

25. Die Verwendung gemäss Anspruch 18 bei Bodendecker-Leguminosen.

**Claims for the Contracting States: DE GB FR CH LI IT NL BE**

1. An N-phenylsulfonyl-N'-pyrimidinyl- or -triazinyl-urea of the general formula I

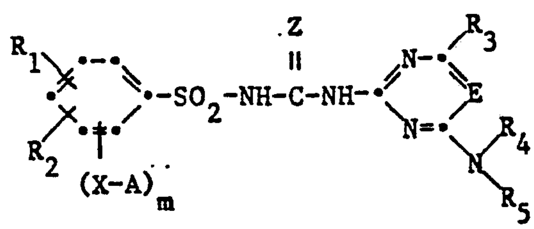

(I),

wherein

A is a $C_3$—$C_6$-alkynyl group, a $C_1$—$C_6$-alkyl group which is substituted by halogen, $C_1$—$C_4$-alkoxy, $C_1$—$C_4$-alkylthio, $C_1$—$C_4$-alkylsulfinyl, $C_1$—$C_4$-alkylsulfonyl, $C_1$—$C_4$-haloalkoxy, $C_1$—$C_4$-haloalkylthio, $C_1$—$C_4$-haloalkylsulfinyl or $C_1$—$C_4$-haloalkylsulfonyl, or it is a $C_2$—$C_6$-alkenyl group substituted by one of the above substituents,

E is the methine group or nitrogen,

X is oxygen, sulfur, or a sulfinyl or sulfonyl bridge,

Z is oxygen or sulfur,

m is the number one or two,

$R_1$ is hydrogen, halogen, $C_1$—$C_5$-alkyl, $C_2$—$C_5$-alkenyl, or a —Y—$R_6$ group,

$R_2$ is hydrogen, halogen, $C_1$—$C_5$-alkyl, $C_2$—$C_5$-alkenyl or $C_1$—$C_4$-haloalkyl, or a —Y—$R_6$, —$COOR_7$, —$NO_2$ or —CO—$NR_8$—$R_9$ group,

$R_3$ is hydrogen, $C_1$—$C_4$-alkyl, $C_1$—$C_4$-alkoxy, $C_1$—$C_4$-alkylthio, $C_1$—$C_4$-haloalkyl, $C_1$—$C_4$-haloalkoxy, halogen or alkoxyalkyl having at most 4 carbon atoms,

$R_4$ is hydrogen, methyl or ethyl,

$R_5$ is hydrogen, $C_1$—$C_4$-alkyl, $C_1$—$C_3$-alkoxy, methoxymethyl, cyanomethyl or cyanoethyl,

$R_6$ and $R_7$ are each $C_1$—$C_5$-alkyl, $C_2$—$C_5$-alkenyl or $C_2$—$C_6$-alkynyl,

$R_8$ and $R_9$ are each independently of the other hydrogen $C_1$—$C_5$-alkyl, $C_2$—$C_5$-alkenyl or $C_2$—$C_6$-alkynyl, and

Y is oxygen, sulfur, or a sulfinyl or sulfonyl bridge,

and A can also be $C_2$—$C_6$-alkenyl when X is oxygen, or a salt of such a compound.

2. A compound according to claim 1, wherein Z is oxygen.

3. A compound according to claim 1, wherein m is the number one.

4. A compound according to claim 1, wherein an —X—A group in the phenyl ring occupies the 2-position with respect to the sulfonamide group.

5. A compound according to claim 1, wherein $R_4$ and $R_5$ together have at most 4 carbon atoms.

6. A compound according to claim 1, wherein X is oxygen.

7. A compound according to claim 1, wherein X and Z are oxygen and m is the number 1, $R_4$ and $R_5$ together have at most 4 carbon atoms, and the —X—A group in the phenyl ring occupies the 2-position with respect to the sulfonamide group.

8. A compound according to claim 7, wherein A is $C_1$—$C_4$-haloalkyl.

9. A compound according to claim 7, wherein A is $C_3$—$C_4$-alkynyl, $C_2$—$C_6$-alkenyl or $C_2$—$C_6$-haloalkenyl, or $C_1$—$C_3$-alkyl substituted by $C_1$—$C_4$-alkoxy, $C_1$—$C_4$-alkylthio, $C_1$—$C_4$-alkylsulfinyl or $C_1$—$C_4$-alkylsulfonyl.

10. A compound according to either one of claims 8 or 9, wherein $R_1$ is hydrogen, and $R_2$ is hydrogen, halogen, methyl or —COO—$C_1$—$C_3$-alkyl.

11. A compound according to claim 10, wherein $R_1$ and $R_2$ are hydrogen.

12. A compound according to claim 11, wherein $R_3$ is methoxy, ethoxy, difluoromethoxy, 2,2,2-trifluoroethoxy, methyl, ethyl, fluoromethyl or chloromethyl, $R_4$ is hydrogen, methyl or ethyl, and $R_5$ is methyl or ethyl.

13. N-(2-Difluoromethoxyphenylsulfonyl)-N'-(4-dimethylamino-6-methoxy-1,3,5-triazin-2-yl)urea according to claim 1.

14. N-(2-Difluoromethoxyphenylsulfonyl)-N'-(4-dimethylamino-6-methoxypyrimidin-2-yl)urea according to claim 1.

15. N-(2-Difluoromethoxyphenylsulfonyl)-N'-(4-methylamino-6-methoxypyrimidin-2-yl)urea according to claim 1.

16. A process for the preparation of a compound of the formula I, claim 1, which process comprises reacting a phenylsulfonamide of the formula II

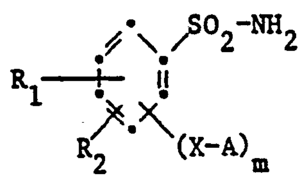

(II),

wherein A, $R_1$, $R_2$, X and m have the meanings defined under the formula I, in the presence of a base, with an N-pyrimidinyl- or -triazinylcarbamate of the formula III

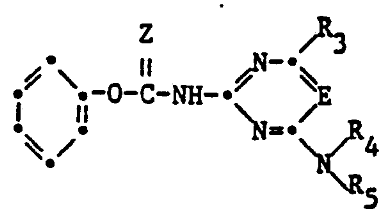

(III),

wherein E, $R_3$, $R_4$, $R_5$ and Z have the meanings defined under the formula I, and, if desired, converting the product obtained into a salt thereof.

17. A process for the preparation of a compound of the formula I, claim 1, which process comprises reacting a phenylsulfonylisocyanate or -isothiocyanate of the formula IV

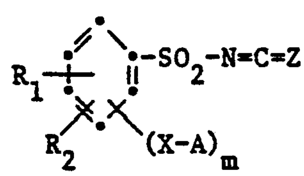

(IV),

wherein A, $R_1$, $R_2$, m, X and Z have the meanings defined under the formula I, in the presence or absence of a base, with an amine of the formula V

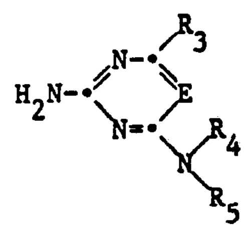

(V),

wherein E, $R_3$, $R_4$ and $R_5$ have the meanings defined under the formula I, and, if desired, converting the product obtained into a salt thereof.

18. A process for the preparation of a compound of the formula I, claim 1, which process comprises reacting a sulfonamide of the formula II indicated in claim 16, in the presence or absence of a base, with an isocyanate or isothiocyanate of the formula VI

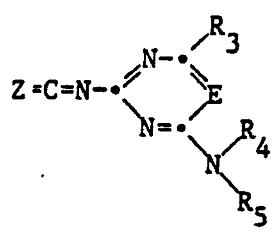

(VI),

wherein E, $R_3$, $R_4$, $R_5$ and Z have the meanings defined under the formula I, and, if desired, converting the product obtained into a salt thereof.

19. A process for the preparation of a compound of the formula I, claim 1, which process comprises reacting an N-phenylsulfonylcarbamate of the formula VII

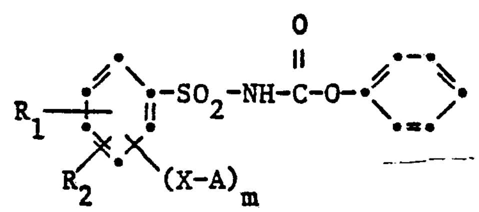

(VII),

wherein A, $R_1$, $R_2$, m and X have the meanings defined under the formula I, with an amine of the formula V indicated in claim 17, and, if desired, converting the product obtained into a salt thereof.

20. A process for the preparation of a compound of the formula I, claim 1, which process comprises reacting a sulfonylurea of the formula VIII

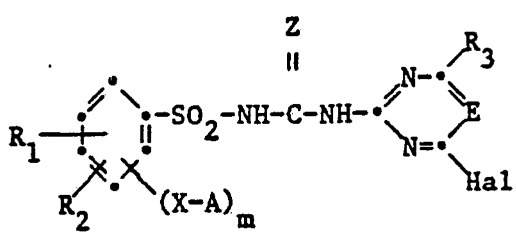

(VIII),

wherein A, E, $R_1$, $R_2$, $R_3$, X, Z and m have the meanings defined under the formula I, and Hal is chlorine or bromine, with an amine of the formula IX

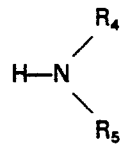

(IX)

wherein $R_4$ and $R_5$ have the meanings defined under the formula I, and, if desired, converting the product obtained into a salt thereof.

21. A process for the preparation of an addition salt of the formula I according to any one of claims 16 to 20, which process comprises reacting a sulfonylurea of the formula I with an amine, an alkali metal hydroxide or alkaline-earth metal hydroxide or a quaternary ammonium base.

22. A herbicidal and plant growth regulating composition which contains, as active ingredient, at least one N-phenylsulfonyl-N'-triazinyl- or -pyrimidinylurea of the formula I, claim 1, together with carriers and/or other additives.

23. A method of combating undesirable plant growth, which method comprises applying thereto or to the locus thereof an effective amount of an N-phenylsulfonyl-N'-triazinyl- or -pyrimidinylurea of the formula I, claim 1, or of a composition containing it as active ingredient.

24. A method of regulating plant growth, which method comprises applying to the plants or to the locus thereof an effective amount of an N-phenylsulfonyl-N'-triazinyl- or -pyrimidinylurea of the formula I, claim 1, or of a composition containing it as active ingredient.

25. A method according to claim 23 of selectively combating weeds pre- or postemergence in crops of useful plants, which method comprises applying thereto or to the locus thereof an effective amount of an N-phenylsulfonyl-N'-triazinyl- or -pyrimidinylurea of the formula I, or of a composition containing it as active ingredient.

26. A method according to claim 24 of suppressing plant growth beyond the two-leaf stage, which method comprises applying pre-emergence an effective amount of an N-phenylsulfonyl-N'-triazinyl- or -pyrimidinylurea of the formula I, or of a composition containing it as active ingredient.

27. A method according to claim 25, wherein the crops are crops of sugar cane, cereals, maize, rice and cotton.

28. A method according to claim 25, wherein the crops are soya-bean crops.

29. A method of regulating the growth of cultivated plants for the purpose of increasing yields, which method comprises applying thereto or to the locus thereof an effective amount of an N-phenylsulfonyl-N'-triazinyl- or -pyrimidinylurea of the formula I, claim 1, or of a composition containing it as active ingredient.

30. A method according to claim 29, wherein the crops are soya-bean crops.

31. A method according to claim 24, wherein the crops are leguminous cover crops.

**Claims for the Contracting State: AT**

1. A herbicidal and plant growth regulating composition which contains, as active ingredient, at least one N-phenylsulfonyl-N'-pyrimidinyl- or -triazinylurea of the general formula I

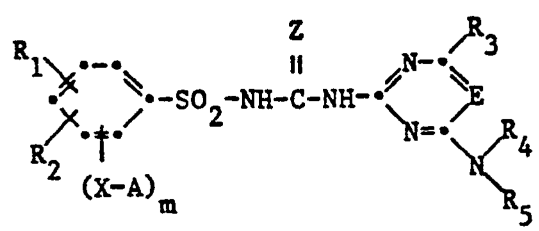

(I),

wherein

A is a $C_3$—$C_6$-alkynyl group, a $C_1$—$C_6$-alkyl group which is substituted by halogen, $C_1$—$C_4$-alkoxy, $C_1$—$C_4$-alkylthio, $C_1$—$C_4$-alkylsulfinyl, $C_1$—$C_4$-alkylsulfonyl, $C_1$—$C_4$-haloalkoxy, $C_1$—$C_4$-haloalkylthio, $C_1$—$C_4$-haloalkylsulfinyl or $C_1$—$C_4$-haloalkylsulfonyl, or it is a $C_2$—$C_6$-alkenyl group substituted by one of the above substituents,

E is the methine group or nitrogen,

X is oxygen, sulfur, or a sulfinyl or sulfonyl bridge,

Z is oxygen or sulfur,

m is the number one or two,

$R_1$ is hydrogen, halogen, $C_1$—$C_5$-alkyl, $C_2$—$C_5$-alkenyl, or a —Y—$R_6$ group,

$R_2$ is hydrogen, halogen, $C_1$—$C_5$-alkyl, $C_2$—$C_5$-alkenyl or $C_1$—$C_4$-haloalkyl, or a —Y—$R_6$, —$COOR_7$, —$NO_2$ or —CO—$NR_8$—$R_9$ group,

$R_3$ is hydrogen, $C_1$—$C_4$-alkyl, $C_1$—$C_4$-alkoxy, $C_1$—$C_4$-alkylthio, $C_1$—$C_4$-haloalkyl, $C_1$—$C_4$-haloalkoxy, halogen or alkoxyalkyl having at most 4 carbon atoms,

$R_4$ is hydrogen, methyl or ethyl,

$R_5$ is hydrogen, $C_1$—$C_4$-alkyl, $C_1$—$C_3$-alkoxy, methoxymethyl, cyanomethyl or cyanoethyl,

$R_6$ and $R_7$ are each $C_1$—$C_5$-alkyl, $C_2$—$C_5$-alkenyl or $C_2$—$C_6$-alkynyl,

$R_8$ and $R_9$ are each independently of the other hydrogen, $C_1$—$C_5$-alkyl, $C_2$—$C_5$-alkenyl or $C_2$—$C_6$-alkynyl, and

Y is oxygen, sulfur, or a sulfinyl or sulfonyl bridge,

and A can also be $C_2$—$C_6$-alkenyl when X is oxygen, or a salt thereof, together with carriers and/or other additives.

2. A composition according to claim 1, wherein Z is oxygen.

3. A composition according to claim 1, wherein m is the number one.

4. A composition according to claim 1, wherein an —X—A group in the phenyl ring occupies the 2-position with respect to the sulfonamide group.

5. A composition according to claim 1, wherein $R_4$ and $R_5$ together have at most 4 carbon atoms.

6. A composition according to claim 1, wherein X is oxygen.

7. A composition according to claim 1, wherein X and Z are oxygen and m is the number 1, $R_4$ and $R_5$ together have at most 4 carbon atoms, and the —X—A group in the phenyl ring occupies the 2-position with respect to the sulfonamide group.

8. A composition according to claim 7, wherein A is $C_1$—$C_4$-haloalkyl.

9. A composition according to claim 7, wherein A is $C_3$—$C_4$-alkynyl, $C_2$—$C_6$-alkenyl or $C_2$—$C_6$-haloalkenyl, or $C_1$—$C_3$-alkyl substituted by $C_1$—$C_4$-alkoxy, $C_1$—$C_4$-alkylthio, $C_1$—$C_4$-alkylsulfinyl or $C_1$—$C_4$-alkylsulfonyl.

10. A composition according to either one of claims 8 or 9, wherein $R_1$ is hydrogen, and $R_2$ is hydrogen, halogen, methyl or —COO—$C_1$—$C_3$-alkyl.

11. A composition according to claim 10, wherein $R_1$ and $R_2$ are hydrogen.

12. A composition according to claim 11, wherein $R_3$ is methoxy, ethoxy, difluoromethoxy, 2,2,2-trifluoroethoxy, methyl, ethyl, fluoromethyl or chloromethyl, $R_4$ is hydrogen, methyl or ethyl, and $R_5$ is methyl or ethyl.

13. A composition according to claim 1, which contains, as active ingredient, N-(2-difluoromethoxyphenylsulfonyl)-N'-(4-dimethylamino-6-methoxy-1,3,5-triazin-2-yl)urea.

14. A composition according to claim 1, which contains, as active ingredient, N-(2-difluoromethoxyphenylsulfonyl)-N'-(4-dimethylamino-6-methoxypyrimidin-2-yl)urea.

15. A composition according to claim 1, which contains, as active ingredient, N-(2-difluoromethoxyphenylsulfonyl)-N'-(4-methylamino-6-methoxypyrimidin-2-yl)urea.

16. A process for the preparation of a compound of formula I, claim 1, which process comprises either

a) reacting a phenylsulfonamide of the formula II

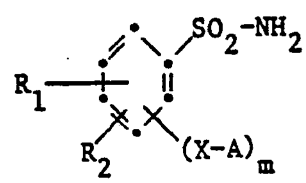

(II),

wherein A, $R_1$, $R_2$, X and m have the meanings defined under the formula I, in the presence of a base, with an N-pyrimidinyl- or -triazinylcarbamate of the formula III

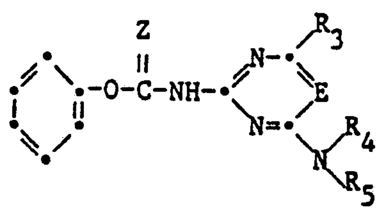

(III),

wherein E, $R_3$, $R_4$, $R_5$ and Z have the meanings defined under the formula I, or

b) reacting a phenylsulfonylisocyanate or -isothiocyanate of the formula IV

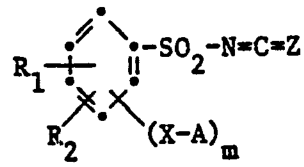

(IV),

wherein A, $R_1$, $R_2$, m, X and Z have the meanings defined under the formula I, in the presence or absence of a base, with an amine of the formula V

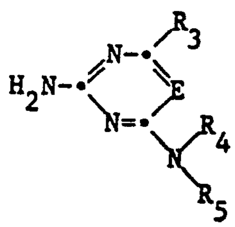

(V),

wherein E, $R_3$, $R_4$ and $R_5$ have the meanings defined under the formula I, or

c) reacting a sulfonamide of the formula II indicated above, in the presence or absence of a base, with an isocyanate or isothiocyanate of the formula VI

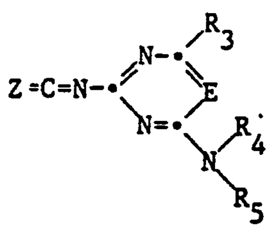

(VI),

wherein E, $R_3$, $R_4$, $R_5$ and Z have the meanings defined under the formula I, or

d) reacting an N-phenylsulfonylcarbamate of the formula VII

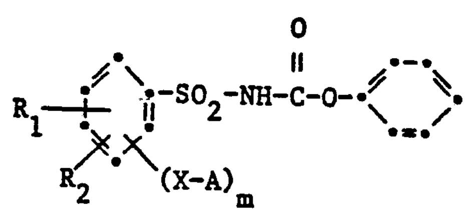

(VII),

wherein A, $R_1$, $R_2$, m and X have the meanings defined under the formula I, with an amine of the formula V indicated above, or

e) reacting a sulfonylurea of the formula VIII

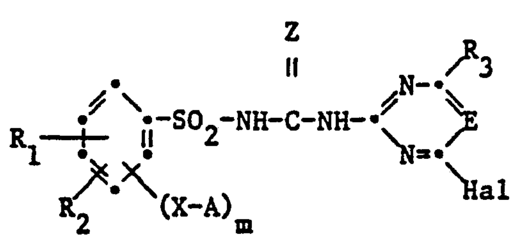

(VIII),

wherein A, E, $R_1$, $R_2$, $R_3$, X, Z and m have the meanings defined under the formula I, and Hal is chlorine or bromine, with an amine of the formula IX

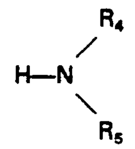

(IX)

wherein $R_4$ and $R_5$ have the meanings defined under the formula I, and, if desired, converting the resultant sulfonylurea of formula I into a salt thereof by reacting said sulfonylurea of formula I with an amine, an alkali metal hydroxide or alkaline earth metal hydroxide or a quaternary ammonium base.

17. A method of combating undesirable plant growth, which method comprises applying thereto or to the locus thereof an effective amount of an N-phenylsulfonyl-N'-triazinyl- or -pyrimidinylurea of the formula I, claim 1, or of a composition containing it as active ingredient.

18. A method of regulating plant growth, which method comprises applying to the plants or to the locus thereof an effective amount of an N-phenylsulfonyl-N'-triazinyl- or -pyrimidinylurea of the formula I, claim 1, or of a composition containing it as active ingredient.

19. A method according to claim 17 of selectively combating weeds pre- or postemergence in crops of useful plants, which method comprises applying thereto or to the locus thereof an effective amount of an N-phenylsulfonyl-N'-triazinyl- or -pyrimidinylurea of the formula I, or of a composition containing it as active ingredient.

20. A method according to claim 18 of suppressing plant growth beyond the two-leaf stage, which method comprises applying pre-emergence an effective amount of an N-phenylsulfonyl-N'-triazinyl- or -pyrimidinylurea of the formula I, or of a composition containing it as active ingredient.

21. A method according to claim 19, wherein the crops are crops of sugar cane, cereals, maize, rice and cotton.

22. A method according to claim 19, wherein the crops are soya-bean crops.

23. A method of regulating the growth of cultivated plants for the purpose of increasing yields, which method comprises applying thereto or to the locus thereof an effective amount of an N-phenylsulfonyl-N'-triazinyl- or -pyrimidinylurea of the formula I, claim 1, or of a composition containing it as active ingredient.

24. A method according to claim 23, wherein the crops are soya-bean crops.

25. A method according to claim 18, wherein the crops are leguminous cover crops.

**Revendications pour les Etats contractants: DE GB FR CH LI IT NL BE**

1. N-phénylsulfonyl-N'-pyrimidinyl- et -triazinyl-urées de formule générale

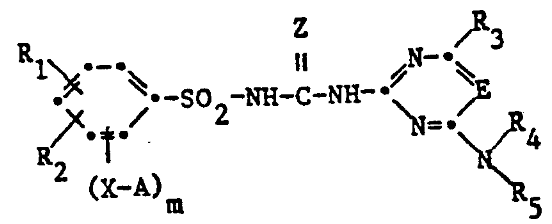

(I),

dans laquelle

A représente un groupe alcynyle en $C_3$—$C_6$, un groupe alkyle en $C_1$—$C_6$ substitué par des halogènes, des groupes alcoxy en $C_1$—$C_4$, alkylthio en $C_1$—$C_4$, alkylsulfinyle en $C_1$—$C_4$, alkylsulfonyle en $C_1$—$C_4$, halogénoalcoxy en $C_1$—$C_4$, halogénoalkylthio en $C_1$—$C_4$, halogénoalkylsulfinyle en $C_1$—$C_4$ ou halogénoalkylsulfonyle en $C_1$—$C_4$, ou bien un groupe alcényle en $C_2$—$C_6$ portant les substituants qu'on vient d'énumérer,

E représente le groupe méthine ou l'azote,

X représente l'oxygène, le soufre, un point sulfinyle ou sulfonyle,

Z représente l'oxygène ou le soufre,

m est égal à 1 ou 2,

$R_1$ représente l'hydrogène, un halogène, un groupe alkyle en $C_1$—$C_5$, alcényle en $C_2$—$C_5$ ou un groupe —Y—$R_6$,

$R_2$ représente l'hydrogène, un halogène, un groupe alkyle en $C_1$—$C_5$, alcényle en $C_2$—$C_5$, halogénoalkyle en $C_1$—$C_4$, ou un groupe —Y—$R_6$, —$COOR_7$, —$NO_2$ ou —CO—$NR_8$—$R_9$,

$R_3$ représente l'hydrogène, un groupe alkyle en $C_1$—$C_4$, alcoxy en $C_1$—$C_4$, alkylthio en $C_1$—$C_4$, halogénoalkyle en $C_1$—$C_4$, halogénoalcoxy en $C_1$—$C_4$, un halogène ou un groupe alcoxyalkyle contenant au maximum 4 atomes de carbone,

$R_4$ représente l'hydrogène, un groupe méthyle ou éthyle,

$R_5$ représente l'hydrogène, un groupe alkyle en $C_1$—$C_4$, alcoxy en $C_1$—$C_3$, méthoxyméthyle, cyanométhyle ou cyanéthyle,

$R_6$ et $R_7$ représentent chacun un groupe alkyle en $C_1$—$C_5$, alcényle en $C_2$—$C_5$ ou alcynyle en $C_2$—$C_6$,

$R_8$ et $R_9$ représentent chacun, indépendamment l'un de l'autre, l'hydrogène, un groupe alkyle en $C_1$—$C_5$, alcényle en $C_2$—$C_5$ ou alcynyle en $C_2$—$C_6$, et

Y représente l'oxygène, le soufre, un pont sulfinyle ou sulfonyle,

A pouvant également représenter un groupe alcényle en $C_2$—$C_6$ lorsque X représente l'oxygène, et les sels de ces composés.

2. Composés selon la revendication 1, caractérisés en ce que Z représente l'oxygène.

3. Composés selon la revendication 1, caractérisés en ce que m est égal à 1.

4. Composés selon la revendication 1, caractérisés en ce qu'un groupe —X—A dans le noyau phényle occupe la position 2 par rapport au groupe sulfonamide.

5. Composés selon la revendication 1, caractérisés en ce que les groupes $R_4$ et $R_5$ contiennent ensemble au maximum 4 atomes de carbone.

6. Composés selon la revendication 1, caractérisés en ce que X représente l'oxygène.

7. Composés selon la revendication 1, caractérisés en ce que X et Z représentent l'oxygène et m est égal à 1, $R_4$ et $R_5$ ensemble ne contiennent pas plus de 4 atomes de carbone et le groupe —X—A dans le noyau phényle occupe la position 2 par rapport au groupe sulfonamide.

8. Composés selon la revendication 7, caractérisés en ce que A représente un groupe halogénoalkyle en $C_1$—$C_4$.

9. Composés selon la revendication 7, caractérisés en ce que A représente un groupe alcynyle en $C_3$—$C_4$, alcényle ou halogénoalcényle en $C_2$—$C_6$ ou un groupe alkyle en $C_1$—$C_3$ substitué par des groupes alcoxy en $C_1$—$C_4$, alkylthio en $C_1$—$C_4$, alkylsulfinyle en $C_1$—$C_4$ ou alkylsulfonyle en $C_1$—$C_4$.

10. Composés selon l'une des revendications 8 ou 9, caractérisés en ce que $R_1$ représente l'hydrogène et $R_2$ représente l'hydrogène, un halogène, un groupe méthyle ou —COO- alkyle en $C_1$—$C_3$.

11. Composés selon la revendication 10, caractérisés en ce que $R_1$ et $R_2$ représentent l'hydrogène.

12. Composés selon la revendication 11, caractérisés en ce que $R_3$ représente un groupe méthoxy, éthoxy, difluorométhoxy, 2,2,2-trifluoréthoxy, méthyle, éthyle, fluorométhyle ou chlorométhyle, $R_4$ représente l'hydrogène, un groupe méthyle ou éthyle et $R_5$ représente un groupe méthyle ou éthyle.

13. La N-(2-difluorométhoxyphényl-sulfonyl)-N'-(4-diméthylamino-6-méthoxy-1,3,5-triazine-2-yl)-urée selon la revendication 1.

14. La N-(2-difluorométhoxyphényl-sulfonyl)-N'-(4-diméthylamino-6-méthoxy-pyrimidine-2-yl)-urée selon la revendication 1.

15. La N-(2-difluorométhoxyphényl-sulfonyl)-N'-(4-méthylamino-6-méthoxy-pyrimidine-2-yl)-urée selon la revendication 1.

16. Procédé de préparation des composés de formule I, revendication 1, caractérisé en ce que l'on fait réagir un phénylsulfonamide de formule II

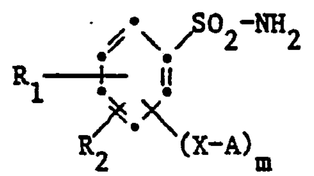

(II),

dans laquelle A, $R_1$, $R_2$, X et M ont les significations indiquées en référence à la formule I, en présence d'une base, avec un N-pyrimidinyl- ou triazinyl-carbamate de formule

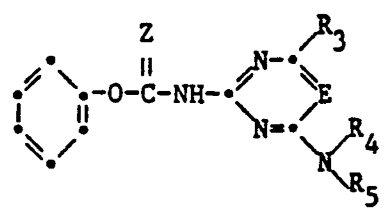

(III),

dans laquelle E, $R_3$, $R_4$, $R_5$ et Z ont les significations indiquées en référence à la formule I, et le cas échéant on convertit le composé obtenu en ses sels.

17. Procédé de préparation des composés de formule I, revendication 1, caractérisé en ce que l'on fait réagir un phénylsulfonylisocyanate ou -isothiocyanate de formule IV

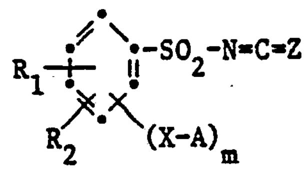

(IV),

dans laquelle A, $R_1$, $R_2$, m, X et Z ont les significations indiquées en référence à la formule I, éventuellement en présence d'une base, avec une amine de formule V

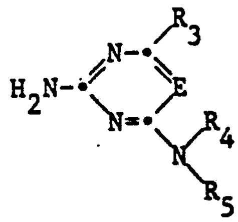

(V),

dans laquelle E, $R_3$, $R_4$ et $R_5$ ont les significations indiquées en référence à la formule I, et le cas échéant on convertit le composé obtenu en ses sels.

18. Procédé de préparation des composés de formule I, revendication 1, caractérisé en ce que l'on fait réagir des sulfonamides de formule II de la revendication 16, éventuellement en présence d'une base, avec un isocyanate ou isothiocyanate de formule VI

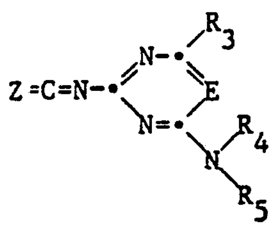

(VI),

dans laquelle E, $R_3$, $R_4$, $R_5$ et Z ont les significations indiquées en référence à la formule I, et le cas échéant on convertit le composé obtenu en ses sels.

19. Procédé de préparation des composés de formule I, revendication 1, caractérisé en ce que l'on fait réagir un N-phénylsulfonylcarbamate de formule VII

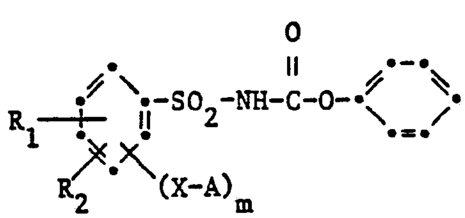

(VII),

dans laquelle A, $R_1$, $R_2$, m et X ont les significations indiquées en référence à la formule I, avec une amine de formule V de la revendication 17 et le cas échéant on convertit le composé obtenu en ses sels.

20. Procédé de préparation des composés de formule I, revendication 1, caractérisé en ce que l'on fait réagir une sulfonylurée de formule VIII

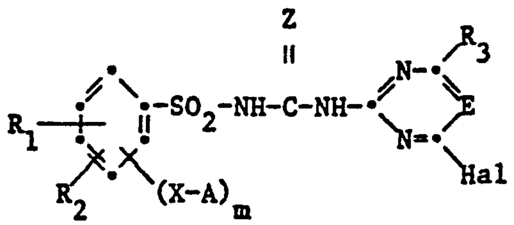

(VIII),

dans laquelle A, E, $R_1$, $R_2$, $R_3$, X, Z et m ont les significations indiquées en référence à la formule I, et Hal représente le chlore ou le brome, avec une amine de formule IX

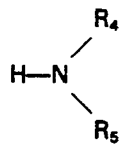

(IX)

dans laquelle $R_4$ et $R_5$ ont les significations indiquées en référence à la formule I, et le cas échéant on convertit le composé obtenu en ses sels.

21. Procédé de préparation des sels formés par addition des composés de formule I selon l'une des revendications 16 à 20, caractérisé en ce que l'on fait réagir une sulfonylurée de formule I avec une amine, un hydroxyde de métal alcalin ou alcalino-terreux ou une base d'ammonium quaternaire.

22. Un produit herbicide et régulateur de la croissance de végétaux, caractérisé en ce qu'il contient en tant que substance active, avec des véhicules et/ou d'autres additifs, au moins une N-phénylsulfonyl-N'-triazinyl- ou -pyrimidinyl-urée de formule I, revendication 1.

23. L'utilisation des N-phénylsulfonyl-N'-triazinyl- ou -pyrimidinyl-urées de formule I, revendication 1, ou de produits en contenant pour la lutte contre les croissances de végétaux indésirables.

24. L'utilisation des N-phénylsulfonyl-N'-triazinyl- ou -pyrimidinyl-urées de formule I, revendication 1, ou de produits en contenant pour la régulation de la croissance des végétaux.

25. L'utilisation des N-phénylsulfonyl-N'-triazinyl- ou -pyrimidinyl-urées de formule I ou de produits en contenant selon la revendication 23, pour la lutte sélective en pré-levée ou en post-levée contre les mauvaises herbes dans les cultures de végétaux utiles.

26. L'utilisation des N-phénylsulfonyl-N'-triazinyl- ou -pyrimidinyl-urées de formule I ou de produits en contenant selon la revendication 24, pour l'inhibition de la croissance des végétaux au-delà du stade deux feuilles, caractérisée en ce que l'on applique les substances actives en pré-levée.

27. L'utilisation selon la revendication 25 dans des cultures de canne à sucre, de céréales, de maïs, de riz et de coton.

28. L'utilisation selon la revendication 25 dans des cultures de soja.

29. L'utilisation des N-phénylsulfonyl-N'-triazinyl- ou -pyrimidinyl-urées de formule I, revendication 1, ou de produits en contenant, pour la régulation de la croissance des végétaux cultivés en vue d'un accroissement de récolte.

30. L'utilisation selon la revendication 29, dans des cultures de soja.

31. L'utilisation selon la revendication 24, sur les légumineuses de couvertures de sol.

**Revendications pour l'Etat contractant: AT**

1. Un produit herbicide et régulateur de la croissance des végétaux, caractérisé en ce qu'il contient en tant que substance active, avec des véhicules et/ou d'autres additifs, au moins une N-phénylsulfonyl-N'-triazinyl- ou -pyrimidinyl-urée de formule I

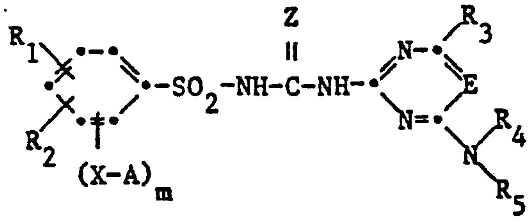

(I),

ou un sel d'un tel composé, dans lesquels

A représente un groupe alcynyle en $C_3$—$C_6$, un groupe alkyle en $C_1$—$C_6$ substitué par des halogènes, des groupes alcoxy en $C_1$—$C_4$, alkylthio en $C_1$—$C_4$, alkylsulfinyle en $C_1$—$C_4$, alkylsulfonyle en $C_1$—$C_4$, halogénoalcoxy en $C_1$—$C_4$, halogénoalkylthio en $C_1$—$C_4$, halogénoalkylsulfinyle en $C_1$—$C_4$ ou halogénoalkylsulfonyle en $C_1$—$C_4$, ou bien un groupe alcényle en $C_2$—$C_6$ portant les substituants qu'on vient d'énumérer,

E représente le groupe méthine ou l'azote,

X représente l'oxygène, le soufre, un point sulfinyle ou sulfonyle,

Z représente l'oxygène ou le soufre,

m est égal à 1 ou 2,

$R_1$ représente l'hydrogène, un halogène, un groupe alkyle en $C_1$—$C_5$, alcényle en $C_2$—$C_5$ ou un groupe —Y—$R_6$,

$R_2$ représente l'hydrogène, un halogène, un groupe alkyle en $C_1$—$C_5$, alcényle en $C_2$—$C_5$, halogénoalkyle en $C_1$—$C_4$, ou un groupe —Y—$R_6$, —$COOR_7$, —$NO_2$ ou —CO—$NR_8$—$R_9$,

$R_3$ représente l'hydrogène, un groupe alkyle en $C_1$—$C_4$, alcoxy en $C_1$—$C_4$, alkylthio en $C_1$—$C_4$, halogénoalkyle en $C_1$—$C_4$, halogénoalcoxy en $C_1$—$C_4$, un halogène ou un groupe alcoxyalkyle contenant au maximum 4 atomes de carbone,

$R_4$ représente l'hydrogène, un groupe méthyle ou éthyle,

$R_5$ représente l'hydrogène, un groupe alkyle en $C_1$—$C_4$, alcoxy en $C_1$—$C_3$, méthoxyméthyle, cyanométhyle ou cyanéthyle,

$R_6$ et $R_7$ représentent chacun un groupe alkyle en $C_1$—$C_5$, alcényle en $C_2$—$C_5$ ou alcynyle en $C_2$—$C_6$,

$R_8$ et $R_9$ représentent chacun, indépendamment l'un de l'autre, l'hydrogène, un groupe alkyle en $C_1$—$C_5$, alcényle en $C_2$—$C_5$ ou alcynyle en $C_2$—$C_6$, et

Y représente l'oxygène, le soufre, un pont sulfinyle ou sulfonyle,

A pouvant également représenter un groupe alcényle en $C_2$—$C_6$ lorsque X représente l'oxygène,

et les sels de ces composés.

2. Produit selon la revendication 1, caractérisé en ce que Z représente l'oxygène.

3. Produit selon la revendication 1, caractérisé en ce que m est égal à 1.

4. Produit selon la revendication 1, caractérisé en ce qu'un groupe —X—A occupe dans le noyau phényle la position 2 par rapport au groupe sulfonamide.

5. Produit selon la revendication 1, caractérisé en ce que les groupes $R_4$ et $R_5$ ont ensemble au maximum 4 atomes de carbone.

6. Produit selon la revendication 1, caractérisé en ce que X représente l'oxygène.

7. Produit selon la revendication 1, caractérisé en ce que X et Z représentent l'oxygène et m est égal à 1, $R_4$ et $R_5$ ensemble, ne contiennent pas plus de 4 atomes de carbone et le groupe —X—A dans le noyau phényle la position 2 par rapport au groupe sulfonamide.

8. Produit selon la revendication 7, caractérisé en ce que A représente un groupe halogénoalkyle en $C_1$—$C_4$.

9. Produit selon la revendication 7, caractérisé en ce que A représente un groupe alcynyle en $C_3$—$C_4$, alcényle ou halogénoalcényle en $C_2$—$C_6$, ou un groupe alkyle en $C_1$—$C_3$ substitué par des groupes alcoxy en $C_1$—$C_4$, alkylthio en $C_1$—$C_4$, alkylsulfinyle en $C_1$—$C_4$ ou alkylsulfonyle en $C_1$—$C_4$.

10. Produit selon l'une des revendications 8 ou 9, caractérisé en ce que $R_1$ représente l'hydrogène et $R_2$ l'hydrogène, un halogène, un groupe méthyle ou —COO-alkyle en $C_1$—$C_3$.

11. Produit selon la revendication 10, caractérisé en ce que $R_1$ et $R_2$ représentent l'hydrogène.

12. Produit selon la revendication 11, caractérisé en ce que $R_3$ représente un groupe méthoxy, éthoxy, difluorométhoxy, 2,2,2-trifluorométhoxy, méthyle, éthyle, fluorométhyle ou chlorométhyle, $R_4$ représente l'hydrogène, un groupe méthyle ou éthyle et $R_5$ représente un groupe méthyle ou éthyle.

13. Produit selon la revendication 1, caractérisé en ce qu'il contient en tant que substance active la N-(2-difluorométhoxyphényl-sulfonyl)-N'-(4-diméthylamino-6-méthoxy-1,3,5-triazine-2-yl)-urée.

14. Produit selon la revendication 1, caractérisé en ce qu'il contient en tant que substance active la N-(2-difluorométhoxyphényl-sulfonyl)-N'-(4-diméthylamino-6-méthoxypyrimidine-2-yl)-urée.

15. Produit selon la revendication 1, caractérisé en ce qu'il contient en tant que substance active la N-(2-difluorométhoxyphényl-sulfonyl)-N'-(4-méthylamino-6-méthoxypyrimidine-2-yl)-urée.

16. Procédé de préparation des composés de formule I, revendication 1, caractérisé en ce que l'on fait réagir

a) un phénylsulfonamide de formule II

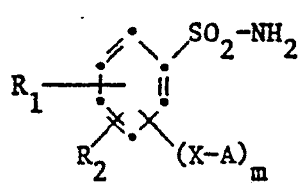

(II),

dans laquelle A, $R_1$, $R_2$, X et M ont les significations indiquées en référence à la formule I, en présence d'une base, avec un N-pyrimidinyl- ou triazinyl-carbamate de formule

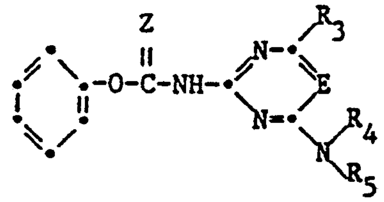

(III),

dans laquelle E, $R_3$, $R_4$, $R_5$ et Z ont les significations indiquées en référence à la formule I, ou bien

b) un phénylsulfonylisocyanate ou -isothiocyanate de formule IV

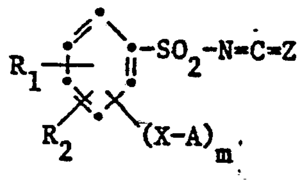

(IV),

dans laquelle A, $R_1$, $R_2$, m, X et Z ont les significations indiquées en référence à la formule I, éventuellement en présence d'une base, avec une amine de formule V

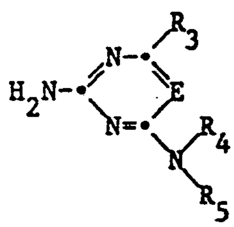

(V),

dans laquelle E, $R_3$, $R_4$ et $R_5$ ont les significations indiquées en référence à la formule I, ou bien

c) des sulfonamides de formule II ci-dessus, éventuellement en présence d'une base, avec un isocyanate ou isothiocyanate de formule VI

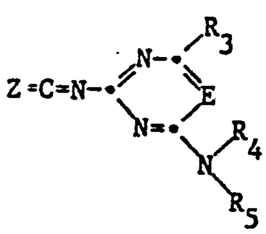

(VI),

dans laquelle E, $R_3$, $R_4$, $R_5$ et Z ont les significations indiquées en référence à la formule I, ou bien

d) un N-phénylsulfonylcarbamate de formule VII

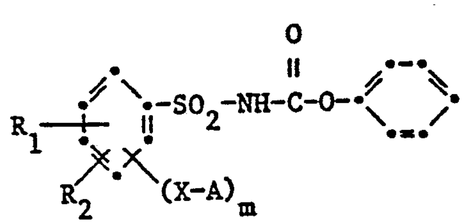

(VII),

dans laquelle A, $R_1$, $R_2$, m et X ont les significations indiquées en référence à la formule I, avec une amine de formule V ci-dessus, ou bien

e) une sulfonylurée de formule VIII

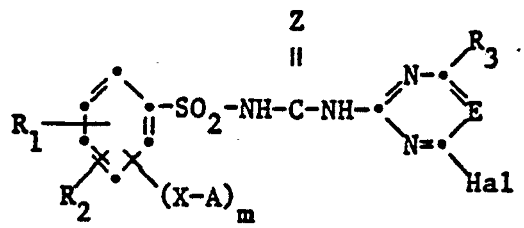

(VIII),

dans laquelle A, E, $R_1$, $R_2$, $R_3$, X, Z et m ont les significations indiquées en référence à la formule I et Hal représente le chlore ou le brome, avec une amine de formule IX

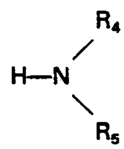

(IX)

dans laquelle $R_4$ et $R_5$ ont les significations indiquées en référence à la formule I, et le cas échéant on convertit les composés obtenus en leurs sels en faisant réagir une sulfonylurée de formule I avec une amine, un hydroxyde de métal alcalin ou alcalino-terreux ou une base d'ammonium quaternaire.

17. L'utilisation des N-phénylsulfonyl-N'-triazinyl- ou -pyrimidinyl-urées de formule I, revendication 1, ou de produits en contenant dans la lutte contre les croissances de végétaux indésirables.

18. L'utilisation des N-phénylsulfonyl-N'-triazinyl- ou -pyrimidinyl-urées de formule I, revendication 1, ou de produits en contenant pour la régulation de la croissance des végétaux.

19. L'utilisation des N-phénylsulfonyl-N'-triazinyl- ou -pyrimidinyl-urées de formule I, ou de produits en contenant selon la revendication 17, pour la lutte sélective en pré-levée ou en post-levée contre les mauvaises herbes dans des cultures de végétaux utiles.

20. L'utilisation des N-phénylsulfonyl-N'-triazinyl- ou -pyrimidinyl-urées de formule I ou de produits en contenant, selon la revendication 18, pour l'inhibition de la croissance des plantes au-delà du stade 2 feuilles, caractérisée en ce que les substances actives sont appliquées en pré-levée.

21. L'utilisation selon la revendication 19, dans les cultures de canne à sucre, de céréales, de maïs, de riz et de coton.

22. L'utilisation selon la revendication 19, dans les cultures de soja.

23. L'utilisation des N-phénylsulfonyl-N'-triazinyl- ou -pyrimidinyl-urées de formule I, revendication 1, ou de produits en contenant pour la régulation de la croissance des végétaux cultivés dans le but d'une augmentation de récolte.

24. L'utilisation selon la revendication 23 dans les cultures de soja.

25. L'utilisation selon la revendication 18 sur les légumineuses de couvertures de sol.